# EUROPÄISCHE PATENTSCHRIFT

(11) **EP 2 895 097 B1**
(45) Veröffentlichungstag und Bekanntmachung des Hinweises auf die Patenterteilung: **03.05.2017**
(21) Anmeldenummer: 13762152.0
(22) Anmeldetag: 16.09.2013
(51) Int. Cl.: A61B 17/02, A61B 17/00, A61B 90/30, A61B 90/35, A61B 90/53

(54) **BELEUCHTUNGSVORRICHTUNG FÜR CHIRURGISCHE ZWECKE**
LIGHTING DEVICE FOR SURGICAL PURPOSES
DISPOSITIF D'ÉCLAIRAGE À USAGE CHIRURGICAL

(30) Priorität: 14.09.2012 DE 102012018170
(43) Veröffentlichungstag der Anmeldung: 22.07.2015
(73) Patentinhaber: Corlife oHG, 30625 Hannover (DE)
(72) Erfinder: HAVERICH, Axel, 30177 Hannover (DE); NOWAK, Kamil, 30519 Hannover (DE); HARDER, Michael, 31141 Hildesheim (DE)
(74) Vertreter: Arth, Hans-Lothar
(86) Internationale Anmeldenummer: PCT/EP2013/069161
(87) Internationale Veröffentlichungsnummer: WO 2014/041172

(56) Entgegenhaltungen:
- WO-A1-02/07632
- WO-A1-2009/137941
- DE-U1-202004 002 963
- JP-A- 2008 034 209
- US-A1- 2007 019 400
- US-A1- 2009 097 236

## Beschreibung

Die Erfindung betrifft eine Beleuchtungsvorrichtung, die für die Befestigung an einem chirurgischen Instrument oder über Adapter an einem Körperteil, insbesondere einem Finger eines Operateurs oder einer/s OP-Assistentin/-en ausgerüstet ist, um während einer chirurgischen Operation, insbesondere innerhalb einer Körperhöhle bzw. Organhöhle genuinem oder traumatischem Ursprungs oder in sonstigen schwer zugänglichen Körpergebieten, als Lichtquelle zu dienen. Dabei ist an dem Gehäuse der Beleuchtungsvorrichtung in einem in der Befestigungsposition zum chirurgischen Instrument gerichteten Wandbereich wenigstens ein von der Wand des Gehäuses nach außen weisender Zapfen angeordnet, der an seinem distalen Ende eine Lichtaustrittsöffnung aufweist und der dafür ausgelegt ist, eine Fensterung des chirurgischen Instruments oder des Adapters zu durchgreifen und so mit der Fensterung eine form- oder kraftschlüssige Verbindung herzustellen und wobei an dem Wandbereich des Gehäuses ein zusätzlicher Befestigungsansatz vorgesehen ist, der zum Einschalten und Ausschalten des im Zapfen angeordneten Leuchtmittels dient. Der Zapfen ist vorzugsweise beweglich ausgestaltet, um eine optimale Ausleuchtung des Operationssitus zu erreichen. Die Erfindung betrifft zudem ein Set aus der Beleuchtungsvorrichtung und mindestens einem chirurgischen Instrument.

### Begriffsdefinitionen

"Chirurgische Instrumente" sind hier alle Instrumente, die von einem Chirurgen, insbesondere für operative Eingriffe, benötigt werden. Hierzu zählen u.a. Retraktoren, allgemein Spreizwerkzeuge (Spreizer), Pinzetten, Skalpelle, Klemmen, Bohrer, Rohrschaftinstrumente, Zangen usw.. Retraktoren sind alle Geräte und Hilfsmittel für das Offenhalten des Situs, wie z.B., aber nicht beschränkt auf, Spreizer, Sperrerblätter (Blades), Rahmen und Wundhaken.

Eine "Fensterung" eines chirurgischen Instruments bezeichnet mindestens ein Durchgangsloch an einem Instrumententeil.

Ein "Set" bezeichnet eine Zusammenstellung mehrerer in Zuordnung zueinander vorliegender Instrumente, Geräte oder Bauteile. Ein "Set" wird häufig auch als "Kit" bezeichnet. Das Set kann sich in einer gemeinsamen Verpackungseinheit für die Einzelteile befinden. In der Operationstechnik ist es auch üblich, dass Sets für bestimmte Operationstechniken zusammengestellt werden. Eine Verpackungseinheit oder ein Kasten, speziell auch ein Sterilkasten, können dabei verschiedenste Instrumente, Ersatzteile und Zusatzteile beinhalten, die für eine bestimmte Operationstechnik benötigt werden.

### Stand der Technik

Traditionell werden zur Ausleuchtung einer chirurgischen Wunde Deckenleuchten, Stirnlampen und Lichtleiter eingesetzt, die jedoch erhebliche Nachteile aufweisen. Deckenleuchten liefern zwar eine hohe Beleuchtungsstärke, können aber den Schattenwurf des im Lichtkegel hantierenden Personals trotz schwenk- und drehbarem Lichtkegelkopf nicht verhindern. Besonders für enge und tiefe Wunden ist eine gute Ausleuchtung durch die Deckenleuchten nicht gegeben. Stirnlampen weisen eine ähnliche Problematik auf, da auch hier das Licht von oben kommt, und haben außerdem den Nachteil, dass nur der Operateur ausreichend sehen kann. Des Weiteren ist die Bewegungsfreiheit des Operateurs aufgrund der Verkabelung vieler Modelle eingeschränkt. Zusätzlich kann das Tragen der meist schweren Stirnleuchte ermüdend wirken. Beide Systeme können zudem, trotz Infrarotfilter, zu einer starken Wärmeentwicklung im Operationssaal führen. Fiberoptische Geräte wie Lichtleiter führen zwar keine Wärme ab, da sie mit einer Kaltlichtquelle ausgestattet sind, und verhindern auch den Schattenwurf durch Instrumente und Köpfe, da sie direkt in-situ zum Einsatz kommen, engen aber das ohnehin oftmals stark eingeschränkte Manipulationsfeld zusätzlich ein und unterliegen einem aufwendigen, teuren und ökologisch bedenklichen Resterilisationsprozess.

Aus der US 2007/0189004 A1 ist eine rohrförmige Leuchte mit Licht emittierender Diode (LED) bekannt, die an medizinische, Labor- und andere Instrumente, insbesondere an Greif- und Schneideinstrumente wie Pinzetten und Scheren seitlich angesteckt werden kann. Die Leuchte wird dabei durch Führungsbuchsen gehaltert, die Teil des Instrumentes sind, so dass die Leuchte nur mit solchen Instrumenten verwendet werden kann. Die Stromversorgung erfolgt über Kabel, die mit bzw. neben den Instrumenten geführt und gehandhabt werden müssen, was die Funktion des Instrumentes beeinträchtigen kann und was umständlich und für den Operateur störend ist. Es besteht insbesondere die Gefahr, dass die mit dem Instrument verbundene Leuchte durch das Eigengewicht der Kabel vom Operationstisch fallen kann. Zudem ist diese Leuchte für die Resterilisation vorgesehen.

DE 20 2004 002963 U1 offenbart ein Beleuchtungselement für ein chirurgisches Instrument mit einer Verbindungseinrichtung zur lösbaren Festlegung des Beleuchtungselements an dem Instrument, um eine Beleuchtungsvorrichtung zu schaffen, die die Handhabung des Instrumentes nicht beeinträchtigt. Hierzu kann die Spannungsquelle direkt in die Beleuchtungsvorrichtung integriert sein. Die Verbindung zum Instrument kann über Clipbügel, Einrastmittel, Magnete, Steckverbindungen usw. erfolgen. Es wird jedoch nicht offenbart, dass das zapfenartige Leuchtmittel eine Fensterung des Instruments durchgreift, so dass das eigentliche Gehäuse und der Punkt der Lichtabstrahlung durch das Instrument getrennt werden.

In US 2007/019400 A1 wird eine Arbeitszange mit einer zylindrischen Beleuchtungsvorrichtung zur Beleuchtung des Arbeitsbereiches offenbart. Der Angelpunkt, in dem die beiden Zangenschenkel miteinander verbunden werden, enthält hierbei eine Bohrung, in die die zylindrische Beleuchtungsvorrichtung derart eingelassen werden kann, dass die Lichtquelle zwischen den Zangenbacken sitzt. Ein wesentlicher Unterschied zu der vorliegenden Erfindun ist, dass die Beleuchtungsvorrichtung in den beschriebenen Ausführungsformen während des Zusammenbaus der Zange integriert wird und dann ein integraler Bestandteil der Zange ist.

In JP 2008 034209 A wird eine Vorrichtung beschrieben, mit der es ermöglicht werden soll, während einer Operation einen lokal begrenzten Bereich zu beleuchten, ohne hierbei das Sichtfeld des Operateurs einzuschränken. Hierbei werden flexible, im Wesentlichen zylindrische Beleuchtungsadapter mit einer distalen Lichtquelle (10) und einer integrierten Batterie offenbart, die in eine entsprechende Halterung (7) einer bandartigen Haltevorrichtung (6) gesteckt werden.

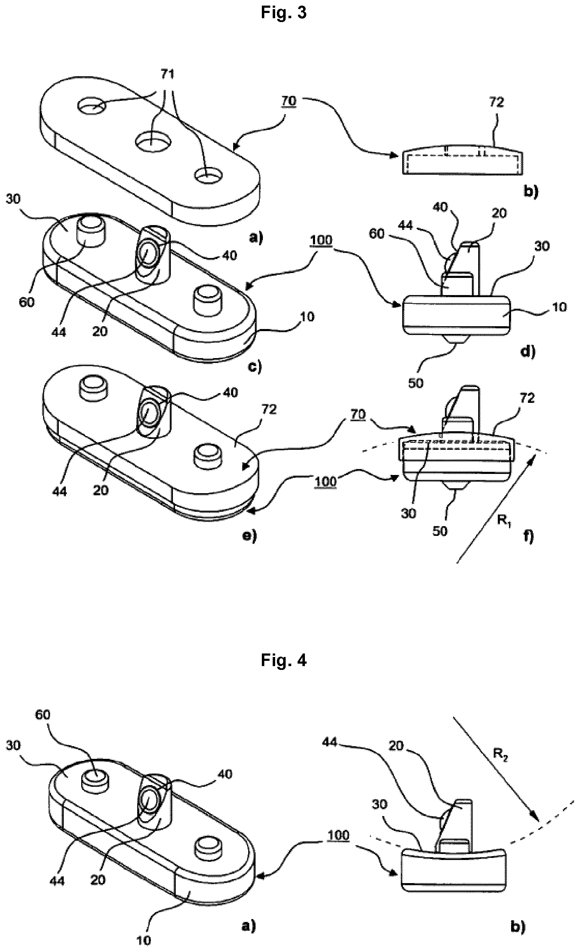

US 2009/0097236 A1 offenbart eine Einweg-Beleuchtungsvorrichtung mit integrierter Spannungsquelle und einer Lichtquelle (LED 22) für medizinische Instrumente (z.B. Speculi), wobei die Lichtquelle in den Kopfteil (14) der Einweg-Beleuchtungsvorrichtung integriert ist und durch verschiedene (mechanische) Schaltertypen an- bzw. ausgeschaltet werden kann. Es ist jedoch nicht offenbart, dass dieses Kopfteil als Zapfen ausgestaltet ist, der eine entsprechende Fensterung der medizinischen Vorrichtung durchgreift und so eigenständig eine kraftschlüssige Verbindung herstellt. Vielmehr zeigt Figur 3, dass die Einweg-Beleuchtungsvorrichtung auf die medizinische Vorrichtung aufgesetzt wird, wobei das Kopfteil inkl. Lichtquelle (besonders die flache Seite (20)) mit einem zweiten flachen Vorsprung (18) zusammen einen Art Greifmechanismus bildet.

In WO 2009/137941 A1 wird eine Beleuchtungsvorrichtung für medizinische Pinzetten offenbart. Die Beleuchtungsvorrichtung ist hierbei im Wesentlichen zylindrisch und enthält eine Lichtquelle, Batterien und Schalter. Die Anbringung an einer Pinzette erfolgt hierbei über einen Clip, der auf die Pinzette geschoben wird und in dessen Klammern wiederum die Beleuchtungsvorrichtung eingeklickt wird. In alternativen Ausführungsformen ist der Clip fester Bestandteil der Beleuchtungsvorrichtung und diese wird auf die Pinzette geschoben. Es wird weder beschrieben noch nahegelegt, dass eine Fensterung eines Instrumentes durch die Beleuchtungsvorrichtung durchgriffen wird, sondern das Instrument durchgreift entweder die Beleuchtungsvorrichtung oder die Beleuchtungsvorrichtung wird auf das Instrument aufgesetzt.

Die Aufgabe der Erfindung besteht darin, die Nachteile im Stand der Technik zu vermeiden und eine vorzugsweise autarke, platzsparende Beleuchtungsvorrichtung zu schaffen, welche die Umgebung des Instruments bzw. den Manipulationsort kontrastreich, farbgetreu und schattenfrei ausleuchtet, ohne den herkömmlichen Operationsablauf zu stören und ohne die Funktion des Instruments zu beeinträchtigen und seine äußere Form wesentlich zu verändern. Es soll sich ferner um eine miniaturisierte Leuchte handeln.

### Beschreibung der Erfindung

Die Aufgabe wird mit Hilfe der Beleuchtungsvorrichtung nach Anspruch 1 und dem Set nach Anspruch 9 gelöst. Vorteilhafte Weiterbildungen der Erfindung sind in den Unteransprüchen, der Beschreibung und den Figuren gekennzeichnet.

Die erfindungsgemäße Beleuchtungsvorrichtung mit einem Gehäuse und wenigstens einem Leuchtmittel ist für die Befestigung an einem chirurgischen Instrument ausgerüstet, um als Lichtquelle während einer chirurgischen Operation zu dienen, wobei das Gehäuse in einem in der Befestigungsposition zum chirurgischen Instrument gerichteten Wandbereich wenigstens einen von der Wand des Gehäuses nach außen weisenden Zapfen aufweist, der
1. an seinem distalen Ende eine Lichtaustrittsöffnung besitzt und
2. dafür ausgelegt ist, eine Fensterung eines chirurgischen Instruments zu durchgreifen und dabei eine form- oder kraftschlüssige Verbindung, wie eine Klemm-, Rast-, Schnapp- oder eine kraftschllüssige bzw. reibschlüssige Steck- oder Pressverbindung herzustellen.

Die Erfindung eignet sich zum Ausleuchten von Brusthöhle oder Bauch, insbesondere für Operationen am Herzen, an der Lunge oder an anderen inneren Organen und für Eingriffe im Bereich der chirurgischen Orthopädie. Die Beleuchtungsvorrichtung eignet sich sowohl für die Anwendung in sterilen Räumlichkeiten, wie beispielsweise in Operationssälen in Krankenhäusern, als auch für Eingriffe in weniger sterilen Umgebungen, wie z.B. in einem Feldlazarett oder in Herstellungseinrichtungen für die Mikroelektronik. Die erfindungsgemäße Beleuchtungsvorrichtung eignet sich weiterhin für den Einsatz in jeglichen Bereichen, wo insbesondere enge und tiefe Öffnungen ausgeleuchtet werden sollen. Der Zapfen der Beleuchtungsvorrichtung steht erfindungsgemäß von einer Gehäusewand ab. Der Zapfen fußt somit auf einer vorzugsweise ebenen Fläche und steht von dieser ab, vorzugsweise im Wesentlichen senkrecht. Der Zapfen bildet hierbei einen Vorsprung, um die Fensterung, die an einem zugeordneten chirurgischen Instrument passend ausgebildet ist, zu durchgreifen und zugleich zur Befestigung zu dienen. Die erfindungsgemäße Konstruktion ermöglicht die Lichtabstrahlung von der Beleuchtungsvorrichtung aus gesehen jenseits der Fensterung. Die Lichtabstrahlung erfolgt von einem gefensterten Instrumententeil aus, wobei die Beleuchtungsvorrichtung selbst hinter diesem Instrumententeil angeordnet ist.

Die vorliegende Erfindung betrifft daher eine Beleuchtungsvorrichtung (100), die für die Befestigung an einem chirurgischen Instrument (200) oder über Adapter (90; 91) an einem Körperteil (92) einer Person, insbesondere an einem Finger, ausgerüstet ist, um als Lichtquelle während einer chirurgischen Operation zur Ausleuchtung von Körper- und Organhöhlen zu dienen, mit einem Gehäuse (10) und wenigstens einem Leuchtmittel, wobei an dem Gehäuse (10) in einem in der Befestigungsposition zum chirurgischen Instrument gerichteten Wandbereich (30) wenigstens ein von der Wand des Gehäuses (10) nach außen weisender Zapfen (20) angeordnet ist, der an seinem distalen Ende eine Lichtaustrittsöffnung (40) besitzt und der dafür ausgelegt ist, eine Fensterung (220) des chirurgischen Instruments oder des Adapters (90; 91) zu durchgreifen und so mit der Fensterung eine form- oder kraftschlüssige Verbindung herzustellen und wobei an dem Wandbereich (30) des Gehäuses (10) ein zusätzlicher Befestigungsansatz (60) in Form eines Stiftes oder einer Noppe vorgesehen ist, der zum Einschalten und Ausschalten des im Zapfen (20) angeordneten Leuchtmittels dient.

Die Länge des Zapfens vom Ansatz auf der Gehäusewand bis zur Unterkante der Lichtaustrittsöffnung beträgt vorzugsweise 5 mm bis 10 mm.

Der Zapfen ist so geformt, dass er eine Fensterung eines chirurgischen Instruments durchgreifen und dabei eine kraft- oder formschlüssige Verbindung eingehen kann, die vorzugsweise als reibschlüssige Steckverbindung oder formschlüssiger Schnapphaken ausgelegt ist. Allgemein ist die Fensterung so ausgelegt, dass in Zusammenwirken mit dem Zapfen eine Klemm-, Rast-, Schnapp-, Steck- oder Pressverbindung verwirklicht wird. Bevorzugt ist der Zapfen im Wesentlichen zylindrisch ausgebildet, was z.B. in Längsrichtung gebogene Zapfen und Zapfen mit abgewandelter Querschnittsform, z.B. eliptischen Querschnitten, mit umfasst.

Für eine reibschlüssige Steckverbindung besitzt der Zapfen vorzugsweise eine zylindrische oder leicht konische Außenwand mit geringem Übermaß zu einer korrespondierenden, die Fensterung bildenden Bohrung an dem chirurgischen Instrument. Im Grunde genommen handelt es sich bei dieser Steckverbindung um eine Passung. Grundsätzlich kann die Steckverbindung durch beliebig geformte Vorsprünge gebildet werden. In der Regel genügt ein geringes Übermaß von wenigen Mikrometern. Die Steckverbindung sollte so ausgelegt sein, dass die Beleuchtungsvorrichtung nur mit einigem Kraftaufwand wieder entfernbar ist, damit sie an dem chirurgischen Instrument sicher gehandhabt werden kann. Vorzugsweise ist die Steckverbindung so ausgelegt, dass sie einer Zugkraft von wenigstens 10 N, weiter vorzugsweise wenigstens 30 N standhält.

Alternativ zu einer Steckverbindung kann eine Rast- oder Schnappverbindung vorgesehen sein, d.h., dass der Zapfen ein Schnappelement, vorzugsweise einen oder mehrere Haken, besitzt, wobei dieses Schnappelement in die Fensterung des chirurgischen Instruments rastend eingreift. Geeignete Rastmechanismen sind dem Fachmann bekannt und brauchen hier nicht näher beschrieben zu werden.

Um die Beleuchtungsvorrichtung von dem Instrument leicht und zerstörungsfrei wieder entfernen zu können, wird eine Steckverbindung bevorzugt. Das chirurgische Instrument kann nach Entfernen der Beleuchtungsvorrichtung resterilisiert werden, während die Beleuchtungsvorrichtung vorzugsweise ein Einwegprodukt ist.

Das Gehäuse der Beleuchtungsvorrichtung ist vorzugsweise aus Kunststoff oder dünnem Metall gefertigt und ist insbesondere kastenförmig oder stabförmig und bevorzugt flach ausgelegt. Die erfindungsgemäße Beleuchtungsvorrichtung ist möglichst klein, so dass die Gehäuselänge bei den derzeit verwendeten Ausführungsformen vorzugsweise maximal 100 mm, weiter vorzugsweise maximal 70 mm, die Gehäusebreite vorzugsweise maximal 50 mm, weiter vorzugsweise maximal 40 mm und die Gehäusehöhe vorzugsweise maximal 25 mm, weiter vorzugsweise maximal 15 mm, beträgt. Bei diesen Abmessungen bleibt das Gewicht für eine Beleuchtungsvorrichtung mit Kunststoffgehäuse und Elektronik vorzugsweise unter 25 g. Das Gehäuse kann mit dem oder den Zapfen einstückig gegossen sein.

Das Gehäuse trägt auf dem zum Instrumententeil gerichteten Wandbereich einen hervorstehenden dünnwandigen Ansatz, der hier als Zapfen (20) bezeichnet wird. Die Funktion des Zapfens wurde vorstehend bereits beschrieben. Im Zapfen befindet sich das optische System der Beleuchtungsvorrichtung. Der Zapfen ist vorzugsweise ein im Wesentlichen zylindrischer Stift, der an seinem distalen Ende eine Lichtaustrittsöffnung aufweist, durch die das Licht des Leuchtmittels austritt und die vorzugsweise mit einer lichtdurchlässigen Abdeckung, aus einem durchsichtigen Kunststoff oder Glas, verschlossen ist. Die Abdeckung ist vorzugsweise als Linse ausgebildet, um die Lichtstreuung zu beeinflussen.

Der Zapfen (20) der Beleuchtungsvorrichtung (100) weist in seinem distalen Ende oder in seinem Kopf das Leuchtmittel hinter der Lichtaustrittsöffnung (40) auf und ist ausgebildet, um in eine Fensterung eines chirurgischen Instruments einzugreifen. Vorzugsweise ist die Fensterung länglich oder als Langloch ausgestaltet, so dass der Zapfen (20) in der länglichen Fensterung oder dem Langloch schiebbar beweglich ist. Um den Situs noch besser ausleuchten zu können ist bei einer besonders bevorzugten Ausführungsform der Zapfen (20) der Beleuchtungsvorrichtung (100) beweglich ausgestaltet, d.h. er kann gebogen und gedreht werden.

Bei dieser besonders bevorzugten Ausführungsform besteht der Zapfen (20) zumindest teilweise aus einem elastischen Kunststoff, so dass der Zapfen (20) manuell gebogen und gedreht werden kann. Die in dem Zapfen (20) von der Stromquelle bis zum Leuchtmittel verlaufenden metallischen Drähte wirken dabei neben ihrer Funktion als elektrische Leiter auch als Seele des Zapfens (20) und wirken den Rückstellkräften des elastischen Kunststoffe entgegen und stabilisieren somit die manuell eingestellt Position des Zapfens (20) oder genauer des Kopfes des Zapfens (20) mit der Lichtaustrittsöffnung (40). Durch diese Beweglichkeit kann der Chirurg den Lichtkegel verstellen und damit den Situs optimal ausleuchten.

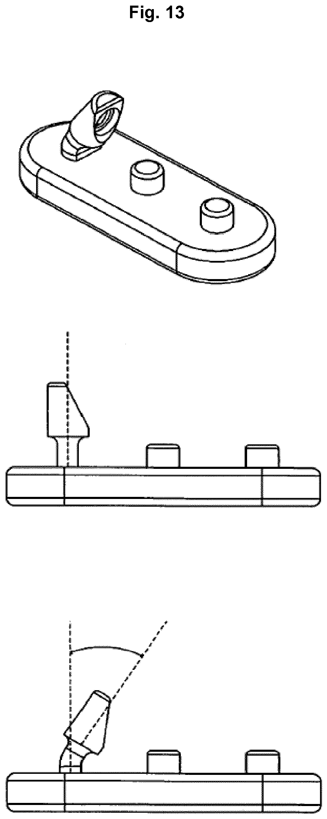

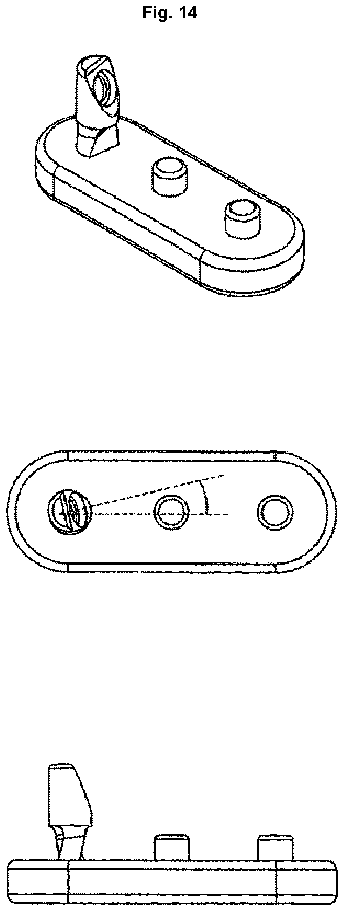

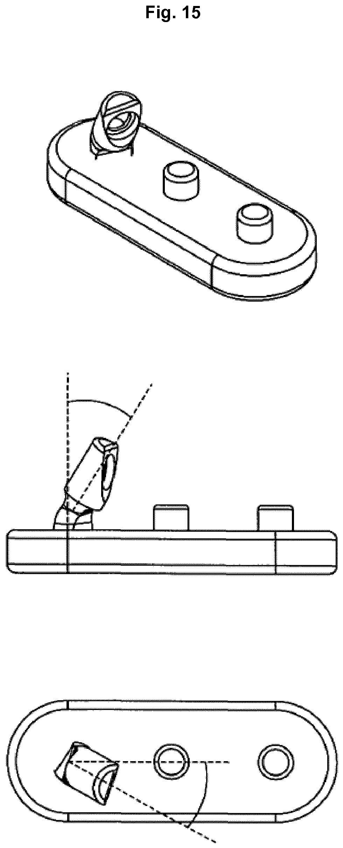

Bevorzugt ist eine Biegbarkeit aus der senkrechten Zentralposition um bis zu 30°, weiter bevorzugt um bis zu 40°, weiter bevorzugt um bis zu 50° und noch weiter bevorzugt um bis zu 60° nach oben als auch um bis zu 30°, weiter bevorzugt um bis zu 40°, weiter bevorzugt um bis zu 50° und noch weiter bevorzugt um bis zu 60° nach unten. Zusätzlich ist eine Drehbarkeit aus der senkrechten Zentralposition um bis zu 30°, weiter bevorzugt um bis zu 40°, weiter bevorzugt um bis zu 50° und noch weiter bevorzugt um bis zu 60° im Uhrzeigersinn als auch eine Drehbarkeit aus der senkrechten Zentralposition um bis zu 30°, weiter bevorzugt um bis zu 40°, weiter bevorzugt um bis zu 50° und noch weiter bevorzugt um bis zu 60° entgegen des Uhrzeigersinns. Natürlich sind auch sämtliche Einstellungen zwischen diesen Maximalwerten möglich, wie beispielsweise eine Drehung um 15° im Uhrzeigersinn und eine Biegung nach oben um 25° bezogen auf die senkrechte Zentralposition. Figur 13 zeigt in der Mitte die senkrechte Zentralposition und in der oberen als auch der unteren Abbildung einen nach unten geneigten Zapfen (20). Figur 14 zeigt einen gedrehten (entgegen des Uhrzeigersinns) aber nicht geneigten Zapfen (20) und Figur 15 zeigen einen gedrehten aus auch geneigten Zapfen (20).

Gemäß einer bevorzugten Ausführungsform befindet sich unter der transparenten Abdeckung bzw. der Linse mindestens eine Leuchtdiode. Der Zapfen ist hohl, um das Leuchtmittel und dessen Zuleitungen aufnehmen zu können. Die im Zapfen angeordneten Komponenten können auch umspritzt bzw. eingegossen sein.

Als Lichtquelle kann grundsätzlich jede Lichtquelle dienen, die eine ausreichende Lichtleistung für die Ausleuchtung des Operationsfeldes bietet. Vorzugsweise weist die Lichtquelle eine Lichtstärke von größer oder gleich 1500 mcd auf. Voraussetzung für den erfindungsgemäßen Einsatz ist, dass die gewünschte Leuchtkraft durch ein Leuchtmittel erreicht wird, das nur so viel Wärme abgibt, dass das umliegende Gewebe nicht unnötig erwärmt wird. Bevorzugt werden daher solche Lichtquellen eingesetzt, die Licht mit sehr geringem Infrarotanteil aussenden (Kaltlichtquellen). Bevorzugt werden Licht emittierende Dioden (LEDs) als Lichtquellen eingesetzt. Vorzugsweise sind eine bis zehn Lichtquellen an einer Beleuchtungsvorrichtung angeordnet, insbesondere ein bis zehn LEDs.

Durch die Verwendung von LEDs als Leuchtmittel wird Infrarotstrahlung vermieden, so dass die Wunde nicht austrocknen kann. Die vom Leuchtmittel an die Leitungen abgegebene und an den Verbrauchern entstehende Wärme ist aufgrund des hohen Wirkungsgrades des Leuchtmittels bei ausreichender Ausleuchtung des Operationsfeldes und aufgrund des geringen Leistungsumsatzes der Schaltung sehr gering, so dass auch eine Erwärmung des Gewebes durch das Gehäuse ausgeschlossen werden kann.

Bei der LED handelt es sich weiter vorzugsweise um eine Weißlichtleuchtdiode. Die Leuchtdiode liegt in SMD-Baugröße (SMD = Surface Mounted Device) vor und ist nur wenige Millimeter groß. Die LED eignet sich hervorragend für Anwendungen im Operationsbereich, da sie aufgrund ihrer lichttechnischen Parameter (CRI > 85 (CRI: *colour rendering index*), Farbtemperatur = 3000 K - 6700 K) die Darstellung von Gewebe und Gewebeflüssigkeit naturgetreu und kontrastreich wiedergibt. Durch diese unverfälschte und originalgetreue Darstellung können verschiedene Gewebetypen gut voneinander unterschieden werden.

Die äußere Form der Beleuchtungsvorrichtung ist in dem Bereich, der in der Befestigungsposition in Kontakt mit dem Instrument kommt oder an das Instrument angrenzt, vorzugsweise an die Form angepasst, die das chirurgische Instrument in dem entsprechenden Ansatzbereich besitzt. In bevorzugter Ausführungsform geschieht dies flächenbündig. Auch wenn der zum chirurgischen Instrument weisende Wandbereich der Beleuchtungsvorrichtung eben ist, kann dies der Kontur des chirurgischen Instruments folgen, wenn diese dort ebenfalls eben sein sollte. Die Beleuchtungsvorrichtung ist in ihrer Form so an das chirurgische Instrument angepasst, dass sie dessen Funktion nicht behindert und dessen äußere Form möglichst wenig beeinträchtigt.

Gemäß einer alternativen Ausführungsform, die nicht der Erfindung entspricht, ist allein ein Zapfen (20) vorgesehen, um die Beleuchtungsvorrichtung an dem chirurgischen Instrument zu befestigen, wobei dieser Zapfen zugleich die einzige Lichtaustrittsöffnung des Leuchtmittels trägt. Alternativ können jedoch auch mehrere Zapfen vorgesehen sein, die mehrere Fensterungen des chirurgischen Instruments durchgreifen, so dass mehrere Lichtaustrittsöffnungen vorhanden sind.

Gemäß der Erfindung ist vorgesehen, dass an dem Wandbereich des Gehäuses zusätzlich zum Zapfen (20) wenigstens ein weiterer Befestigungsansatz, insbesondere eine Noppe oder ein runder Schnapphakenträger, in Zuordnung zu einer passenden Aufnahme an dem chirurgischen Instrument vorgesehen ist. Die Aufnahme kann eine Fensterung sein, also eine Durchgangsöffnung, die auch für einen Zapfen geeignet wäre, oder eine beliebige andere passende Aufnahme, wie z.B. eine Ausfräsung. Eine Ausfräsung benennt hier einen nicht durchgängigen, d.h. die Wand des Instruments nicht durchdringenden, sonstig geformten und verlaufenden Schlitz oder ein Sackloch.

Die zusätzlichen Befestigungsansätze dienen dazu, die Verbindung zwischen Beleuchtungsvorrichtung und chirurgischem Instrument mechanisch noch fester zu gestalten und die Verdrehsicherheit zu erhöhen, was insbesondere bei einem zylindrischen Befestigungszapfen zweckmäßig sein kann.

Auch kann die Verbindungsfestigkeit durch weitere zusätzliche Maßnahmen erhöht werden. So können z.B. haftende Beschichtungen auf den aneinandergrenzenden Flächen von Beleuchtungsvorrichtung und chirurgischem Instrument ausgebildet sein oder die Beleuchtungsvorrichtung kann zusätzlich mit einem Klebstoff an dem Instrument befestigt werden. In bevorzugter Weiterbildung ist vorgesehen, dass es sich dabei um einen Haftklebstoff handelt, der nach Gebrauch der Beleuchtungsvorrichtung leicht wieder abgezogen werden kann.

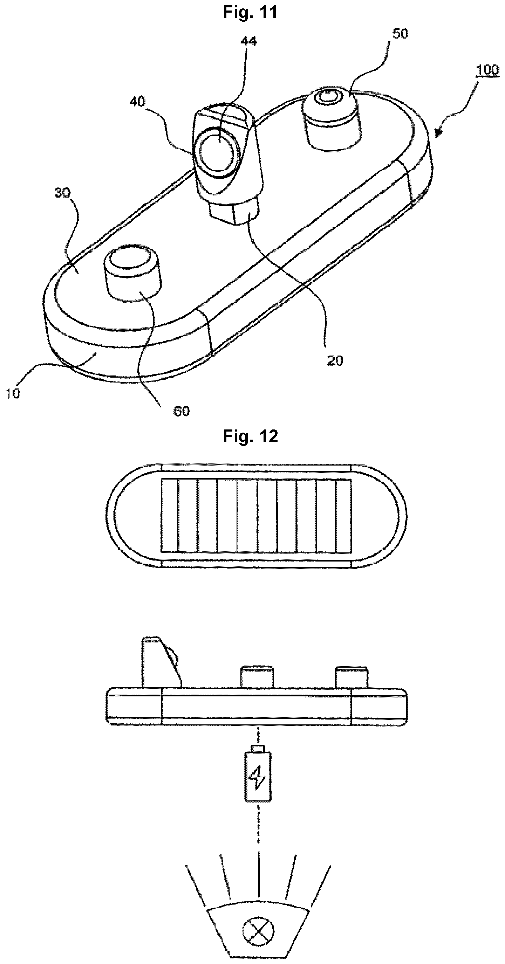

Bei der erfindungsgemäßen Ausführungsform der Beleuchtungsvorrichtung (100) ist mindestens ein weiterer Befestigungsansatz (60) vorhanden, wobei einer dieser weiteren Befestigungsansätze (60) als Schalter ausgestaltet ist, um das Leuchtmittel ein- und aus zu schalten oder neben dem Ein- und Ausschalten auch dessen Lichtstärke bzw. Helligkeit einzustellen. Figur 11 zeigt eine Beleuchtungsvorrichtung (100) mit einem Zapfen (20) in der Mitte und einem vorderen Befestigungsansatz (60) und einem hinteren Befestigungsansatz (60), wobei der hintere Befestigungsansatz (60) als Schalter ausgestaltet ist. Der hintere Befestigungsansatz (60) besteht vorzugsweise aus elastischem Kunststoff oder ist vorzugsweise mit elastischem Kunststoff überzogen und ist vorzugsweise als Druckschalter ausgestaltet. Beispielsweise kann durch einmaliges Drücken dieses Schalters das Leuchtmittel eingeschaltet und durch nochmaliges Drücken wieder ausgeschaltet werden. Bevorzugt ist, wenn durch einmaliges Drücken das Leuchtmittel eingeschaltet und durch wiederholtes Drücken dieses Schalters die Helligkeit stufenweise bis zu einem Maximum gesteigert und durch fortgesetztes Betätigen dieses Schalters die Lichtstärke wieder stufenweise reduziert wird bis zum vollständigen Ausschalten. Bei einer anderen Ausgestaltung dieses Schalters wird durch andauerndes Drücken zuerst das Leuchtmittel eingeschaltet und danach dessen Helligkeit so lange stufenlos gesteigert wie der Schalter betätigt bleibt und bei Erreichung der maximalen Helligkeit die Lichtstärke wieder stufenlos reduziert solange der Schalter betätigt wird bis zum Ausschalten des Leuchtmittels. Bei dieser Ausführungsform können die Funktionen des Einschaltens und Ausschaltens bzw. zusätzlich der Einstellung der Helligkeit als auch der Fixierung der Beleuchtungsvorrichtung (100) am chirurgischen Instrument elegant über einen zusätzlichen als Schalter ausgestalteten Befestigungsansatz (60) verwirklicht werden.

Somit betrifft die vorliegende Erfindung_eine Beleuchtungsvorrichtung (100), die für die Befestigung an einem chirurgischen Instrument (200) oder über Adapter (90; 91) an einem Körperteil (92) einer Person, insbesondere an einem Finger, ausgerüstet ist, wobei die Beleuchtungsvorrichtung (100) ein Gehäuse (10) und wenigstens ein Leuchtmittel aufweist, wobei an dem Gehäuse (10) in einem in der Befestigungsposition zum chirurgischen Instrument gerichteten Wandbereich (30) wenigstens ein von der Wand des Gehäuses (10) nach außen weisender Zapfen (20) und ein Befestigungsansatz (60) angeordnet sind, wobei der Zapfen (20) an seinem distalen Ende eine Lichtaustrittsöffnung (40) besitzt und ein Leuchtmittel enthält und der Zapfen (20) dafür ausgelegt ist, eine Fensterung (220) des chirurgischen Instruments oder des Adapters (90; 91) zu durchgreifen und so mit der Fensterung eine form- oder kraftschlüssige Verbindung herzustellen und der Befestigungsansatz (60) als Schalter zum Ein- und Ausschalten des Leuchtmittels ausgestaltet ist.

Die Beleuchtungsvorrichtung enthält als Energieversorgungseinheit mindestens einen Akkumulator, eine Batterie oder eine Kombination beider Energieträger. In derzeit bevorzugter Ausführungsform ist ein Akkumulator so ausgelegt, dass eine Leuchtdauer von wenigstens 60 Minuten, weiter bevorzugt von 120 Minuten, besonders bevorzugt von mehreren Stunden, gewährleistet ist. Vorzugsweise wird das nahende Ende der Betriebszeit des Akkumulators oder der Batterie durch ein optisches Signal angezeigt. Zudem ist bevorzugt, wenn durch eine Steuerung innerhalb der Beleuchtungsvorrichtung (100) ein erneutes Einschalten nur innerhalb einer vordefinierten Zeitdauer möglich ist, damit eine Wiederverwendung der Beleuchtungsvorrichtung (100) nach einer bestimmten Zeit nicht mehr möglich ist. Durch die interne Energieversorgungseinheit wird eine externe Kabelführung überflüssig und dadurch bedingte Gefahrenquellen werden beseitigt.

Bei einer insbesondere bevorzugten Ausführungsform der vorliegenden Erfindung wird die interne Energieversorgungseinheit, beispielsweise eine Batterie oder ein Akkumulator, über eine am Gehäuse (10) der Beleuchtungsvorrichtung (100) angeordnete Solarzelle aufgeladen. Diese Ausführungsform weist gleich mehrere Vorteile auf. Zum einen werden keine Kabel benötigt, welche von außen in die Beleuchtungsvorrichtung (100) führen, um die interne Energieversorgungseinheit aufzuladen, welche hinderlich sind und zudem ein Sterilitätsproblem darstellen und den Innenraum der Beleuchtungsvorrichtung (100) evtl. nicht dicht abschließen und zum anderen kann eine steril verpackte Beleuchtungsvorrichtung (100) selbst nach längerer Lagerung vor der Auslieferung an z.B. ein Krankenhaus nochmals vollständig aufgeladen werden, damit die interne Energieversorgungseinheit bei dem Einsatz der Beleuchtungsvorrichtung (100) die maximale Kapazität aufweist.

Dies ist ein enormer Vorteil, weil die Beleuchtungsvorrichtungen (100) in steril verpackter Weise gelagert werden und beispielsweise bei einer Lagerung von einem Jahr sich die interne Energieversorgungseinheit halb oder weitgehend vollständig entladen könnte. Bei einer kabelgebundenen Aufladung der internen Energieversorgungseinheit müsste die sterile Verpackung geöffnet und nach dem Aufladevorgang der internen Energieversorgungseinheit die Beleuchtungsvorrichtung (100) erneut steril verpackt werden.

Die Aufladung der internen Energieversorgungseinheit über eine im Gehäuse (10) der Beleuchtungsvorrichtung (100) integrierte Solarzelle kann hingegen durch die sterile Verpackung hindurch erfolgen, ohne dass die sterile Verpackung geöffnet werden müsste.

Normalerweise wird die Beleuchtungsvorrichtung (100) in einer Blisterverpackung steril verpackt und vorzugsweise in einer Sterilverpackung (beispielsweise einem Primärblister aus Thermoform-Folie) und einer Schutzverpackung (beispielsweise einem Sekundärblister aus ähnlichen Materialien wie der Primärblister) verpackt. Nach der Verpackung im Sterilraum in eine oder zwei Blisterverpackungen erfolgt die Sterilisation vorzugsweise mittels Wasserstoffperoxidplasma.

Die Bestrahlung der im Gehäuse (10) der Beleuchtungsvorrichtung (100) integrierten Solarzelle kann durch die Blisterverpackung(en) hindurch erfolgen. Die Blisterverpackung(en) sind durchlässig für sichtbares Licht oder allgemeiner für die Strahlung, welche die Solarzelle zur Erzeugung von elektrischem Strom benötigt, so dass eine kabelfreie Aufladung der internen Energieversorgungseinheit mittels durch die Sterilverpackung hindurch bestrahlter Solarzelle erfolgen kann.

Somit betrifft in einer insbesondere bevorzugten Ausführungsform die vorliegende Erfindung eine Beleuchtungsvorrichtung (100) in einer Sterilverpackung, wobei die Beleuchtungsvorrichtung (100) eine interne Energieversorgungseinheit besitzt und in das Gehäuse (10) der Beleuchtungsvorrichtung (100) zumindest eine Solarzelle integriert ist und die Sterilverpackung den Durchtritt von Strahlung zulässt, welche die Solarzelle zur Erzeugung von elektrischem Strom benötigt.

In anderen Worten betrifft die besonders bevorzugte Ausführungsform eine Beleuchtungsvorrichtung (100) in einer Sterilverpackung, wobei die Beleuchtungsvorrichtung (100) eine interne Energieversorgungseinheit und ein Leuchtmittel besitzt und in das Gehäuse (10) der Beleuchtungsvorrichtung (100) zumindest eine Solarzelle integriert ist, welche zur Aufladung der internen Energieversorgungseinheit dient und die Sterilverpackung den Durchtritt von Strahlung zulässt, welche die Solarzelle zur Erzeugung von elektrischem Strom benötigt, um die interne Energieversorgungseinheit aufzuladen.

Eine weitere bevorzugte Ausführungsform ist gerichtet auf eine Beleuchtungsvorrichtung (100), die für die Befestigung an einem chirurgischen Instrument (200) oder über Adapter (90; 91) an einem Körperteil (92) einer Person, insbesondere an einem Finger, ausgerüstet ist, wobei die Beleuchtungsvorrichtung (100) ein Gehäuse (10) und wenigstens ein Leuchtmittel aufweist, dadurch gekennzeichnet, dass in das Gehäuse (10) mindestens eine Solarzelle integriert ist und an dem Gehäuse (10) in einem in der Befestigungsposition zum chirurgischen Instrument gerichteten Wandbereich (30) wenigstens ein von der Wand des Gehäuses (10) nach außen weisender Zapfen (20) und ein Befestigungsansatz (60) angeordnet ist, der an seinem distalen Ende eine Lichtaustrittsöffnung (40) besitzt und der dafür ausgelegt ist, eine Fensterung (220) des chirurgischen Instruments oder des Adapters (90; 91) zu durchgreifen und so mit der Fensterung eine form- oder kraftschlüssige Verbindung herzustellen und der Befestigungsansatz (60) als Schalter zum Ein- und Ausschalten des Leuchtmittels ausgestaltet ist. Die Solarzelle dient dabei zur Aufladung einer internen Energieversorgungseinheit innerhalb des Gehäuses wie beispielsweise einer Batterie oder eines Akkumulators. Das Leuchtmittel befindet sich zudem vorzugsweise innerhalb des Zapfens (20) bzw. vorzugsweise innerhalb des Zapfens (20) und hinter der Lichtaustrittsöffnung (40).

Bei allen hierin beschriebenen Ausführungsformen ist der Zapfen (20) sowie die Befestigungsansätze (60) dicht mit dem Gehäuse (10) der Beleuchtungsvorrichtung (100) verbunden und die Lichtaustrittsöffnung (40) im Zapfen (20) ist dicht verschlossen beispielsweise mit einer Linse oder einem lichtdurchlässigen Material wie z.B. Glas oder Kunststoff, so dass keine Flüssigkeit und vorzugsweise auch kein Gas in das Innere der Beleuchtungsvorrichtung (100) eindringen kann.

Eine weiter bevorzugte Ausführungsform ist gerichtet auf eine Beleuchtungsvorrichtung (100), die für die Befestigung an einem chirurgischen Instrument (200) oder über Adapter (90; 91) an einem Körperteil (92) einer Person, insbesondere an einem Finger, ausgerüstet ist, wobei die Beleuchtungsvorrichtung (100) ein Gehäuse (10), eine interne Energieversorgungseinheit und wenigstens ein Leuchtmittel aufweist, wobei in das Gehäuse (10) mindestens eine Solarzelle integriert ist und an dem Gehäuse (10) in einem in der Befestigungsposition zum chirurgischen Instrument gerichteten Wandbereich (30) wenigstens ein von der Wand des Gehäuses (10) nach außen weisender Zapfen (20) und ein Befestigungsansatz (60) angeordnet sind, wobei der Zapfen (20) an seinem distalen Ende eine Lichtaustrittsöffnung (40) besitzt und ein Leuchtmittel enthält und der Zapfen (20) dafür ausgelegt ist, eine Fensterung (220) des chirurgischen Instruments oder des Adapters (90; 91) zu durchgreifen und so mit der Fensterung eine form- oder kraftschlüssige Verbindung herzustellen und der Befestigungsansatz (60) als Schalter zum Ein- und Ausschalten des Leuchtmittels ausgestaltet ist und die Solarzelle zur Aufladung der internen Energieversorgungseinheit dient.

Zudem ist insbesondere bevorzugt, wenn die hierin offenbarten Ausführungsformen einer Beleuchtungsvorrichtung (100) mit Solarzelle sich in einer Sterialverpackung befinden.

Das Leuchtmittel der Beleuchtungsvorrichtung wird vorzugsweise über das Heranführen eines externen Magneten an einen im Gehäuse verbauten Magnetschalter, dem Fachmann auch als Reed-Kontakt oder Reed-Schalter bekannt, aktiviert.

Alternativ kann am Gehäuse der Beleuchtungsvorrichtung, wie bei Leuchten üblich, ein per Hand betätigbarer Schalter angeordnet sein, mit dem das oder die Leuchtmittel mit der Stromversorgung verbunden oder von dieser wieder getrennt werden. Eine Kombination aus Magnetschalter und per Hand betätigbarem Schalter ist ebenfalls möglich.

Bevorzugt werden Druckschalter oder Taster verwendet, die in besonders bevorzugter Ausführungsform so auf den Wandbereich des Gehäuses angeordnet sind, der dem chirurgischen Instrument zugewandt ist, dass der Druckschalter oder Taster beim Befestigen der Beleuchtungsvorrichtung durch Kontakt mit dem angrenzenden Instrumententeil betätigt wird. Diese Ausführungsform ist ganz besonders geeignet für eine Beleuchtungsvorrichtung für den einmaligen Gebrauch. Zugleich mit der Befestigung wird die Beleuchtungsvorrichtung eingeschaltet und leuchtet, bis sie wieder entfernt wird oder bis die Energieversorgung versiegt.

Die erfindungsgemäße Beleuchtungsvorrichtung kann mit zusätzlichen Komponenten ausgerüstet sein. Hierzu zählen insbesondere
1. ein Ausgleichselement zwischen Wandbereich des Gehäuses der Beleuchtungsvorrichtung und Wandbereich des chirurgischen Instruments, welches
   a) die Adaptionsfähigkeit der Beleuchtungsvorrichtung an unebene Instrumentenoberflächen ermöglicht bzw. verbessert und
   b) die Festigkeit der Verbindung zwischen chirurgischem Instrument und Beleuchtungsvorrichtung erhöht, um ein Abbrechen der Zapfen oder der Befestigungsansätze bei Belastungen, beispielsweise durch Druck- oder Scherkräfte, zu verhindern;
2. ein Adapter, der die Befestigung der Beleuchtungsvorrichtung an einem Körperteil des Operateurs oder einer/s OP-Assistentin/-en, wie beispielsweise an einem Finger, erlaubt.

Das Ausgleichselement dient in erster Linie dazu, zwischen dem ebenen Gehäusewandbereich der Beleuchtungsvorrichtung und der uneben geformten, gewölbten oder gekehlten Oberfläche eines chirurgischen Instruments einen Ausgleich zu schaffen. Ausgleichselemente können vorzugsweise ein oder mehrere Federbügel, eine Membranhülle oder ein elastisches oder weiches Füllmaterial, z.B. aus Schaumstoff umfassen. Die Ausgleichselemente können zusätzliche Funktionen erfüllen. Eine Membranhülle schützt die Vorrichtung beispielsweise zugleich vor Feuchtigkeit.

Eine bevorzugte Ausführungsform des Ausgleichselements ist ein Federbügel. Der Federbügel besteht vorzugsweise aus mehreren metallischen, längs und quer verlaufenden Blattfedern und kann auf den Wandbereich des Gehäuses der Beleuchtungsvorrichtung rastend, schnappend oder klebend angebracht werden. Beim Aufsetzen der Beleuchtungsvorrichtung auf beispielsweise ein Sperrerblatt werden die Blattfedern entsprechend der Ausformung der Oberfläche dieses Instruments ausgelenkt und an diese angepasst und verbleiben in einer angespannten Position, so dass die Beleuchtungsvorrichtung auch auf unebene Blattoberflächen montierbar ist. Die angespannten Blattfedern wirken dann durch ihre zurückfedernden Eigenschaften und ihre vorgespannte Lagerung den auf die Beleuchtungsvorrichtung wirkenden Kräften, beispielsweise
Gewebekräften, entgegen und fixieren die Beleuchtungsvorrichtung sicher an der Rückseite des Sperrerblattes. Federbügel werden bevorzugt gemeinsam mit rastenden oder anderen formschlüssigen Befestigungsansätzen und/oder Zapfen verwendet, um dem Federdruck besser standzuhalten.

Eine weitere bevorzugte Ausführungsform des Ausgleichselements ist eine Membranhülle. Die Membranhülle besteht aus einem elastischen und weichen Kunststoff. Die Anpassung an die Oberfläche eines bestimmten chirurgischen Instruments erfolgt über die Gestaltung der Oberfläche des Wandbereiches der Membranhülle, so dass diese mit der Oberfläche des Instruments korrespondiert. Die Montage der Membranhülle an die Beleuchtungsvorrichtung erfolgt vorzugsweise durch eine Schnapp-, Rast- oder Klebeverbindung.

Der Adapter zur Befestigung der erfindungsgemäßen Beleuchtungsvorrichtung an einem Körperteil oder einem sonstigen Teil oder Gegenstand kann auf verschiedene Weise ausgebildet sein.

Gemäß einem ersten Ausführungsbeispiel ist vorgesehen, dass der Adapter die Form eines Adapterrings besitzt. Der Adapter ist dabei ähnlich einem Fingerring ausgeführt, der sich mit einem ringförmigen oder spangenförmigen Teil um einen Finger stecken lässt und der, auf dem Ringteil befestigt, eine Aufnahme für die Beleuchtungsvorrichtung trägt. Diese Aufnahme kann die Form eines offenen, geschlitzten Kastens besitzen, in den die Beleuchtungsvorrichtung eingeschoben werden kann.

Gemäß einem weiteren Ausführungsbeispiel kann der Adapter bandförmig ausgebildet sein, wobei es sich vorzugsweise um ein Klettband oder ein Band mit Klettbandenden handelt, das passende Aufnahmen für die Beleuchtungsvorrichtung besitzt. In bevorzugter Ausführungsform besitzt das Klettband wenigstens eine Durchgangsöffnung, durch die der Zapfen der Beleuchtungsvorrichtung von innen nach außen gesteckt wird, wonach das Band um einen Finger oder einen anderen für die Befestigung geeigneten Gegenstand gewickelt und verschlossen wird.

Weitere zusätzliche Komponenten und Vorrichtungsbestandteile sind nicht ausgeschlossen und können zusätzlich an der Beleuchtungsvorrichtung angebracht werden.

Die Aufgabe der Erfindung wird weiterhin durch ein Set gelöst, in dem wenigstens eine Beleuchtungsvorrichtung nach der Erfindung und ein oder mehrere chirurgische Instrumente und/oder Instrumententeile vorhanden sind, von denen wenigstens eines für eine Verbindung mit der Beleuchtungsvorrichtung gefenstert ist. In demselben Set können weitere Instrumente oder Instrumententeile, Zusatzteile, Ersatzteile und Hilfsmittel vorliegen. Besonders bevorzugt ist ein Set, das eine Zusammenstellung für eine bestimmte Operationstechnik enthält. Dem Operateur werden so alle erforderlichen Mittel für die Durchführung einer bestimmten Operationstechnik an die Hand gegeben. Bei dem Set kann es sich vorzugsweise um eine Verpackungseinheit handeln.
In Weiterbildung der Erfindung kann das Set zusätzlich wenigstens einen Adapter für die Fixierung der Beleuchtungsvorrichtung an einem Körperteil und/oder ein Ausgleichselement für den Kontakt zwischen Wand der Beleuchtungsvorrichtung und Instrumentenwand enthalten.

Die mit der Erfindung erzielten Vorteile liegen in der zielgenauen Ausleuchtung des Operationsfeldes ohne dass Körperteile oder Gegenstände in den Lichtweg eingreifen und so einen Schattenwurf auf das Operationsgebiet erzeugen, wie es bei herkömmlichen Operationsleuchten der Fall ist, da sich die Beleuchtungsvorrichtung nach der Erfindung in der Wunde befindet und da lediglich der durch die Fensterung durchgreifende und Licht abgebende Zapfen der auf der Rückseite des chirurgischen Instruments angebrachten Beleuchtungsvorrichtung nur wenige Millimeter in die Wunde ragt.

Ein besonderer Vorteil der Beleuchtungsvorrichtung liegt darin, dass das Operationsfeld auch bei kleinen Wundöffnungen optimal ausgeleuchtet werden kann, da die Beleuchtungsvorrichtung wenig Platz beansprucht, da die Hauptbestandteile der Beleuchtungsvorrichtung wie Gehäuse mit Leuchtmittel, Schalter, Leitungen, Steuerung und Akkumulator heutzutage in miniaturisierter Form verfügbar sind. Eine Einengung des Operationsfeldes findet nicht statt, da die Beleuchtungsvorrichtung auf der Rückseite eines chirurgischen Instruments angeordnet wird, so dass keine ihrer Teile dauerhaft den Operationszugang versperren.

Neben den kleinen geometrischen Dimensionen hat die Beleuchtungsvorrichtung nur ein geringes Gewicht, so dass sie nach der Befestigung auf dem chirurgischen Instrument, auch aufgrund ihrer Positionierung auf der Rückseite, die Funktion des chirurgischen Instruments und den Operationsablauf nicht stört.

Ein weiterer Vorteil der Beleuchtungsvorrichtung besteht darin, dass sie als Einmalartikel ausgelegt sein kann. wodurch eine kosten- und zeitintensive Resterilisation und damit auch der mehrfache Einsatz von gefährlichen und ökologisch bedenklichen Gasen und Chemikalien umgangen wird. Die Beleuchtungsvorrichtung nach der Erfindung wird zudem aus recycelbaren Materialien gefertigt und enthält gemäß der Richtlinie 2011/65/EU (RoHS2) keine elektronischen Komponenten mit gefährlichen oder schädlichen Stoffen.

Die Beleuchtungsvorrichtung ist mit wenigen Handgriffen montierbar, auch innerhalb des Operationsverlaufs.

### Figurenbeschreibung

- Fig. 1: eine nicht zur Erfindung gehörende Befestigungsvorrichtung mit kastenförmigem Gehäuse a) perspektivisch, b) perspektivisch mit gedrehtem Zapfen (Winkel β), c) in Seitenansicht mit magnetischem Schalter, d) in Teilseitenansicht mit Druckschalter auf der Gehäuserückseite, e) in Teilseitenansicht mit Drucktaster oder Druckschalter auf dem Wandbereich der Gehäusevorderseite.
- Fig. 2: ein Ausführungsbeispiel mit Magnetschalter und Verdrehschutznoppe a) perspektivisch, b) in Seitenansicht.
- Fig.3: das Ausführungsbeispiel aus Figur 2. c) perspektivisch und d) in Seitenansicht ergänzt um eine Membranhülle: a) perspektivisch, b) in Seitenansicht, zusammengesteckt: e) perspektivisch und f) in Seitenansicht.
- Fig. 4: ein weiteres Ausführungsbeispiel mit angepasst geformter Vorderwand a) perspektivisch, b) in Seitenansicht.
- Fig. 5: ein Sperrerblatt mit angesetzter Beleuchtungsvorrichtung mit Druckschalter auf der Gehäuserückseite und aufgesetzter Membranhülle als Zwischenstück, a) perspektivisch von vorne, b) perspektivisch von hinten, c) in Vorderansicht.
- Fig. 6: ein Sperrerblatt mit schlitzförmiger Fensterung und aufgesetzter Beleuchtungsvorrichtung mit Membranhülle als Zwischenstück, a) perspektivisch, b) in Vorderansicht.
- Fig. 7: ein weiteres Ausführungsbeispiel für die Beleuchtungsvorrichtung mit aufgesetztem Federbügel als Ausgleichsvorrichtung zur besseren Adaption an ein Sperrerblatt in a) perspektivischer Ansicht, b) in Teilseitenansicht vor der Installation an ein Blade, c) in Teilseitenansicht nach der Installation an ein Blade.
- Fig. 8: ein weiteres Ausführungsbeispiel für ein Sperrerblatt mit aufgesetzter Beleuchtungsvorrichtung mit veränderter Zapfenanordnung in a) perspektivischer Ansicht, b) in Seitenansicht, c) von oben.
- Fig. 9: ein nicht zur Erfindung gehörendes Beispiel der Beleuchtungsvorrichtung in Verbindung mit einem Adapterring für die Befestigung an einem menschlichen Finger a) als Explosionszeichnung und b) in perspektivischer Ansicht.
- Fig. 10: ein nicht zur Erfindung gehörendes Beispiel der Beleuchtungsvorrichtung in Verbindung mit einem Adapterband mit Klettverschluss für die Befestigung an einem menschlichen Finger a) als Explosionszeichnung und b) in perspektivischer Ansicht.
- Fig. 11: zeigt eine bevorzugte Ausführungsform der Beleuchtungsvorrichtung (100) mit einem Zapfen (20) und zwei weiteren Befestigungsansätzen (60), wobei ein Befestigungsansatz (60) gleichzeitig als Schalter ausgestaltet ist, um das Leuchtmittel einzuschalten und auszuschalten als auch optional dessen Lichtstärke (Helligkeit oder Helligkeitsstufen) zu regulieren.
- Fig. 12: zeigt eine bevorzugte Ausführungsform der Beleuchtungsvorrichtung (100) mit einem Zapfen (20) und zwei weiteren Befestigungsansätzen (60) und einer Solarzelle an der Unterseite des Gehäuses (10) der Beleuchtungsvorrichtung (100). Die Solarzelle ist in das transparente Gehäuse integriert (Fig. 12 oben). Die Solarzelle dient zur Aufladung eines Akkumulators oder einer Batterie innerhalb des Gehäuses (10), der bzw. die dann wiederum als Stromquelle für das Leuchtmittel im Kopf des Zapfens (20) dient. In Fig. 12 unten ist eine Ladeleuchte mit emittierter Strahlung gezeigt, welche auf die Solarzelle gerichtet ist, welche dann Strom zum Aufladen der internen Energieversorgungseinheit erzeugt.
- Fig. 13: zeigt eine bevorzugte Ausführungsform der Beleuchtungsvorrichtung (100) mit beweglichem Zapfen (20). Die Abbildung in der Mitte zeigt links den Zapfen (20) in senkrechter Zentralposition, d.h. die Achse durch den Zapfen (20) steht senkrecht auf der Ebene, in der die Oberfläche des Gehäuses (10) liegt und die Lichtaustrittsöffnung (40) weist entlang der Längsachse durch das Gehäuse (10) der Beleuchtungsvorrichtung (100). In der oberen Abbildung in Figur 13 ist der nach unten (bzw. nach vorne) gebogene Zapfen (20) perspektivisch dargestellt. In der unteren Abbildung in Figur 13 ist die Biegung des Zapfens (20) um ca. 45° dargestellt. Der Zapfen (20) ist nur nach unten bzw. nach vorne gebogen aber nicht zusätzlich gedreht.
- Fig. 14: zeigt aus verschiedenen Blickwinkeln einen um ca. 10° entgegen des Uhrzeigersinns gedrehten Zapfens (20). Der Zapfen (20) ist nur gedreht aber nicht gleichzeitig auch gebogen.
- Fig. 15: zeigt aus verschiedenen Blickwinkeln einen nach unten bzw. nach vorne gebogenen als auch im Uhrzeigersinn gedrehten Zapfen (20).

### BEISPIELE

Im Folgenden wird die Erfindung anhand von Beispielen erläutert, die in den Figuren dargestellt sind.

Dies soll allein dem besseren Verständnis der Erfindung dienen, ohne dass die Erfindung auf die gezeigten Beispiele beschränkt wäre. Weitere Beispiele kann der Fachmann aufgrund seines allgemeinen Fachwissens mit Hilfe der vorausgegangenen Beschreibung auffinden.

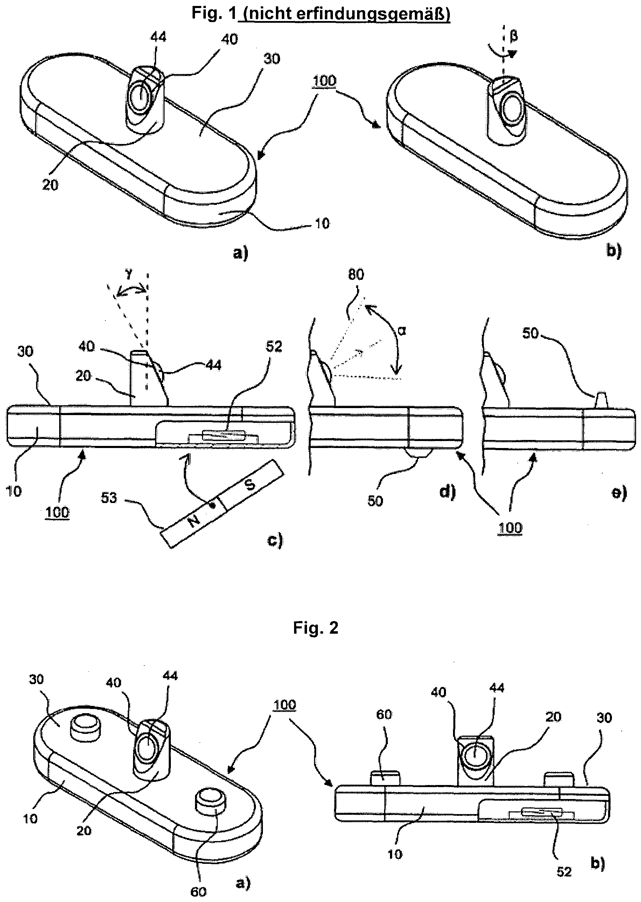

Fig. 1 zeigt ein nicht zur Erfindung gehörendes Beispiel einer Beleuchtungsvorrichtung **100** mit einem kastenförmigen Gehäuse **10,** das einen hier zylindrisch und abgeschrägt ausgebildeten Zapfen **20** trägt. Das Gehäuse **10** ist hier gemeinsam mit dem Zapfen **20** und der lichtdurchlässigen Abdeckung **44** aus einem biokompatiblen Kunststoff hergestellt. Das Gehäuse **10** besitzt einen Wandbereich **30,** der bei Benutzung der Beleuchtungsvorrichtung **100** an einem chirurgischen Instrument wenigstens mit Teilen seiner Außenkontur an einem Instrumententeil anliegt. Innerhalb der vom Wandbereich **30** begrenzten Fläche ist der Zapfen **20** angeordnet, der als Vorsprung vom Gehäuse **10** eine Fensterung am nicht dargestellten Instrument erfordert. In der perspektivischen Ansicht gemäß Fig. 1a) ist zu erkennen, dass der Zapfen **20** mit der Lichtaustrittsöffnung **40** mittig in Bezug auf die Gehäusevorderseite mit dem Wandbereich **30** angeordnet ist. Aus der Seitenansicht gemäß Fig. 1c) ist zu erkennen, dass die Vorderwand des Gehäuses **10** mit dem Wandbereich **30** eben ausgebildet ist. Durch die zylindrische Form des Zapfens **20** klemmt dieser optimal per Reibschluss sowohl in kreisförmigen Bohrungen als auch in Langlöchern, die am nicht dargestellten Instrument als Fensterungen ausgebildet sein können, oder in sonstig am Instrument vorhandenen Durchgangsöffnungen. Im dargestellten Ausführungsbeispiel ist die Lichtaustrittsöffnung **40** durch eine lichtdurchlässige Abdeckung bzw. eine Linse **44** verschlossen, hinter welcher sich eine LED befindet. Die Linse **44** bildet einen Aufsatz auf dem Zapfen **20,** der für gute Lichtstreuung in die Umgebung sorgt. Der Lichtaustrittswinkel für dieses Beispiel ist mit alpha (α) gekennzeichnet. In Fig. 1c) ist zu sehen, dass die Ebene der; Lichtaustrittsöffnung durch eine Anschrägung des Zapfens gegenüber der Zapfenachse um den Winkel gamma (γ) gekippt sein kann, wodurch das Licht gezielter in den Operationsbereich gelenkt wird. Die perspektivische Ansicht in Fig. 1b) zeigt die Beleuchtungsvorrichtung **100** mit einen um den Winkel β verdrehten Zapfen **20** für längliche Modelle von Sperrerblättern. Fig. 1) zeigt außerdem einen Magneten **53,** der beim Heranführen an das Gehäuse **10** einen in der Beleuchtungsvorrichtung **100** platzierten Magnetschalter **52** aktiviert und somit das hier nicht sichtbare Leuchtmittel einschaltet, dessen Lichtstrahl **80** im Winkel α durch die lichtdurchlässige Abdeckung **44** aus der Beleuchtungsvorrichtung tritt.

In Fig. 1d) ist außerdem ein mit einer Membran verschlossener Druckschalter **50** zu erkennen, der - in Bezug auf die auf der Vorderseite angeordnete Lichtaustrittsöffnung **40** am Zapfen **20** - an der Rückseite des Gehäuses **10** angeordnet ist. Mit Hilfe dieses Schalters wird die montierte Beleuchtungsvorrichtung von Hand eingeschaltet. Das Ausführungsbeispiel gemäß Fig. 1e) besitzt einen Tastschalter **50,** der neben dem Zapfen **20** innerhalb der Fläche des Wandbereichs **30** angeordnet ist. Bei diesem Ausführungsbeispiel schließt der Wandbereich **30** flächenbündig an ein ebenes Sperrerblatt an, so dass der Tastschalter **50** beim Befestigen der Beleuchtungsvorrichtung **100** an dem nicht dargestellten chirurgischen Instrument automatisch eingedrückt und die Beleuchtung somit eingeschaltet wird.

Bei dem gezeigten, nicht zur Erfindung gehörendes Beispiel handelt es sich um eine im Kleinformat herstellbare, einfache Beleuchtungsvorrichtung mit nur einem Zapfen und einer Lichtquelle, nämlich einer durch eine Linse **44** abgedeckte Leuchtdiode, die an dem nicht dargestellten chirurgischen Instrument angebracht werden kann, wenn hierfür an einem dünnen Instrumententeil, wie beispielsweise einem Sperrerblatt, das nicht dicker als die Höhe des Zapfens **20** sein darf, eine passende Fensterung ausgebildet ist.

In den meisten Figuren ist bis auf den Magnetschalter **52** das Innere der Beleuchtungsvorrichtung nicht dargestellt. Darin befindet sich jeweils die Energieversorgung mittels Batterie und/oder Akkumulator, einschließlich zugehöriger Leitungen zum Leuchtmittel und zum Schalter sowie andere Schaltungskomponenten.

Fig. 2 zeigt ein Ausführungsbeispiel, das in Fig. 2a) perspektivisch von vorne und in Fig. 2b) von der Seite dargestellt ist. Das Gehäuse **10** der Beleuchtungsvorrichtung **100** ist wie in Fig. 1a) und 1c) geformt, der auf der Vorderseite des Gehäuses **10** als Klemmvorsprung angebrachte Zapfen **20** ist ebenfalls zylindrisch ausgebildet und mit einer lichtdurchlässigen Abdeckung **44** versehen. Die Abdeckung **44** ist hier eine einfache Sammellinse, so dass der Austrittswinkel alpha (α) des Lichtkegels **80** in diesem Beispiel kleiner ist als in Fig. 1 gezeigt, was bei gleich bleibendem Lichtstrom die Beleuchtungsstärke erhöht. Dies bewirkt eine gezielte und starke Ausleuchtung der beleuchteten Operationsfläche. Die Ausführungsform nach Fig. 2 weist zusätzlich zwei Befestigungsansätze **60** in Form von Noppen als Verdrehschutz und als zusätzlichen Befestigungsmechanismus auf. Die Noppen **60** greifen in eine oder mehrere Aussparung/-en des nicht dargestellten Instrumententeils ein, während der Zapfen **20** eine Fensterung des Instrumententeils durchgreift, wobei es sich wiederum um eine kreisrunde Bohrung oder ein Langloch handeln kann. Der Durchmesser des Zapfens **20** ist mit geringem Übermaß zur Bohrung bzw. zur Weite des Langlochs am Instrument ausgebildet. Die Noppen **60** können ebenfalls einen leicht zur Bohrung des chirurgischen Instruments vergrößerten Durchmesser besitzen und als Passung mit geringem Übermaß ausgelegt sein. Das Ausführungsbeispiel gemäß Fig. 2 besitzt zudem einen in Fig. 2b) sichtbaren internen Magnetschalter **52,** der durch das Anlegen eines hier nicht dargestellten Magnetfeldes, beispielsweise eines Magneten **53,** die Beleuchtungsvorrichtung **100** einschaltet.

Fig. 3 zeigt die Beleuchtungsvorrichtung **100** aus Fig. 2, und zwar als solche nochmals in Fig. 3c) perspektivisch und in Fig. 3d) von der Seite. Fig. 3a) und Fig. 3b) zeigen a) perspektivisch und b) von der Seite) eine zugehörige Membranhülle **70,** die auf die Leuchte **100** aufgesteckt werden kann, wie es in Fig. 3e) perspektivisch und in Fig. 3f) von der Seite gezeigt ist. Diese Beleuchtungsvorrichtung trägt einen rückseitigen Schalter **50,** der von Hand oder mit einem spitzen Gegenstand, wie beispielsweise einer Pinzette, bedient werden kann. Die Membranhülle dient als Ausgleichselement, welches die Adaption der ebenen Fläche des Wandbereichs **30** dieser Ausführungsvariante an eine abgerundete Rückseite eines Sperrerblattes ermöglicht. Die Anpassung an ein bestimmtes Sperrerblattmodell wird dabei über die Auslegung des Radius R₁ des Wandbereichs **72** der Membranhülle **70** erreicht, der dem Wölbungsradius des jeweiligen Sperrerblattes entspricht. Die Membranhülle **70** ist mit Fensterungen **71** ausgestattet, die mit dem Zapfen **20** und den Noppen **60** der Beleuchtungsvorrichtung korrespondieren, was die Montage der Membranhülle auf die Beleuchtungsvorrichtung **100** erlaubt. Die Membranhülle wird dabei nur aufgesetzt und schnappt oder rastet seitlich am Gehäuse **10** ein. In einer anderen, hier nicht aufgeführten Ausführungsform, kann die Verbindung von Membranhülle und Gehäuse **10** auch klebend erfolgen.

Fig. 4 zeigt ein anderes abgewandeltes Ausführungsbeispiel, bei dem die Wand **30** des Gehäuses **10** der Beleuchtungsvorrichtung **100** nach innen gewölbt ist. Diese Beleuchtungsvorrichtung **100** kann beispielsweise von innen an dem Griff einer Zange befestigt werden, wobei die äußere Form der Beleuchtungsvorrichtung **100** durch die den Zapfen **20** tragende und nach innen gewölbte Vorderfläche an die Form einer Auswölbung im Instrumentenschenkel angeglichen ist und flächenbündig an dieser Auswölbung angreift. Der eingezeichnete Radius R₂ des äußeren Wandbereichs **30** des Gehäuses **10** der Beleuchtungsvorrichtung **100** ist dabei genau an die Rückseite der ausgewölbten Oberfläche eines chirurgischen Instruments angepasst.

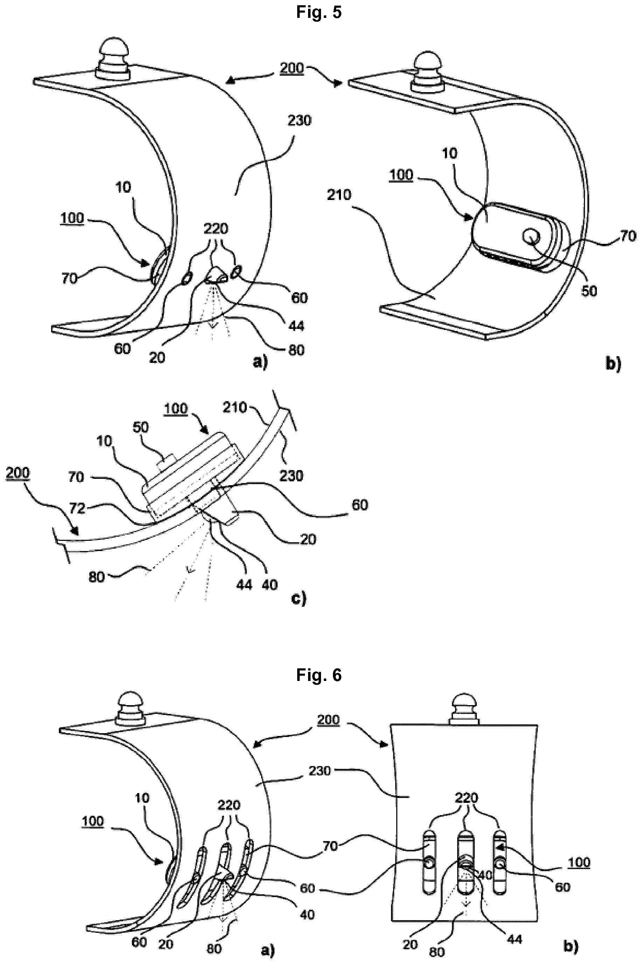

Fig. 5 zeigt ein Sperrerblatt **200** eines im Übrigen nicht dargestellten chirurgischen Instruments mit einer auf der Rückseite oder Innenseite **210** des Sperrerblattes befindlichen Beleuchtungsvorrichtung **100.** Der Zapfen **20** der Beleuchtungsvorrichtung greift durch eine als runde Durchgangsöffnung ausgeführte Fensterung **220** des Sperrerblattes **200.** Da die Rückseite des dargestellten Sperrerblattes **210** gerundet ist und die Beleuchtungsvorrichtung in dieser Ausführung einen ebenen vorderen Wandbereich **30** besitzt, wie in Fig.1 und Fig. 2, wird als Ausgleich eine Membranhülle **70** zwischen der Oberfläche der Rückseite des Blattes **210** und dem Wandbereich **30** der Beleuchtungsvorrichtung **100** angebracht. In Fig. 5c) ist gut zu erkennen, dass die elastische Membranhülle **70** sich mit ihrer Oberfläche **72** sowohl an die Gehäuseform **10,** wie auch die Rückseite **210** des Sperrerblattes anschmiegt. Die Membranhülle **70** ist aus einem elastischen Kunststoffmaterial gefertigt. Der Zwischenraum zwischen dem Wandbereich **30** des Gehäuses **10** und der Rückseite **210** des Sperrerblattes wird durch das Membranmaterial ausgefüllt bzw. abgedichtet. In diesem Ausführungsbeispiel sind der Wandbereich **30** der Beleuchtungsvorrichtung **100** und der ihm zugeordnete, in der Befestigungsposition an die Beleuchtungsvorrichtung **100** angrenzende Bereich des chirurgischen Instrumententeils nicht gegengleich ausgebildet und nicht flächenbündig angeordnet, was durch die Membranhülle **70** ausgeglichen wird. Die Membranhülle **70** umschließt die Beleuchtungsvorrichtung **100** zugleich feuchtigkeitsdicht, sie kann optional einen hohen Reibungskoeffizienten haben und haftend wirken oder sie kann klebend oder haftklebend ausgerüstet sein. In diesem Ausführungsbeispiel erfolgt die Aktivierung der Beleuchtungsvorrichtung **100** über einen am Gehäuse **10** angebrachten mechanischen Druckschalter **50.** Der Zapfen **20** trägt eine lichtdurchlässige Abdeckung **44,** unter der sich eine LED befindet, deren Lichtabstrahlung durch gestrichelte Linien in Fig. 5c) verdeutlicht ist. Das gezeigte Sperrerblatt **200** besitzt insgesamt drei Fensterungen **220,** die mittlere für den Durchgang des Zapfens **20** und zwei seitliche Bohrungen, durch die zwei Noppen **60** greifen, die als Klemmvorsprünge für zusätzlichen Halt der Beleuchtungsvorrichtung **100** sorgen. Alternativ können die Fensterungen **220** des Sperrerblattes **200** so bemessen sein, dass nur die Noppen **60** eine Klemmverbindung zwischen Beleuchtungsvorrichtung **100** und Sperrerblatt **200** herstellen, während der Zapfen **20** durch die zugehörige Fensterung **220** nur durchgesteckt ist. Für diese Steckverbindung des Zapfens **20** wird er ohne Übermaß oder mit leichtem Untermaß im Vergleich zur Fensterung **220** hergestellt. Dies schont den Zapfen. Da die Klemmverbindung durch die Noppen **60** bereitgestellt wird, muss nicht noch der Zapfen **20** mit der Linse **44** kraftschlüssig in die zugehörige Fensterung **220** eingepresst werden. Alternativ verfügt das Sperrerblatt über ein Vielfaches der benötigten Fensterungen, die in einigem Abstand zueinander positioniert sind, so dass die Beleuchtungsvorrichtung **100** je nach Bedarf platziert werden kann.

Fig. 6 zeigt ein entsprechendes Ausführungsbeispiel wie in Fig. 5, bei dem jedoch die Fensterungen **220** als Langlöcher ausgeführt sind. Die Beleuchtungsvorrichtung **100** gleicht der aus Fig. 5. Fig. 6b) zeigt das Ausführungsbeispiel, das in Fig. 6a) perspektivisch dargestellt ist, in der Vorderansicht. Die Fixierung der Noppen **60** und gegebenenfalls zusätzlich des Zapfens **20** erfolgt gleitend durch die Langlöcher. Die Abstrahlrichtung des Leuchtmittels kann hierdurch verändert werden. Diese Ausleuchtungsjustierung kann der Operateur vor Ort dadurch vornehmen, dass er die Beleuchtungsvorrichtung **100** an der gewünschten Position innerhalb der Langlöcher der Fensterungen **220** anordnet.

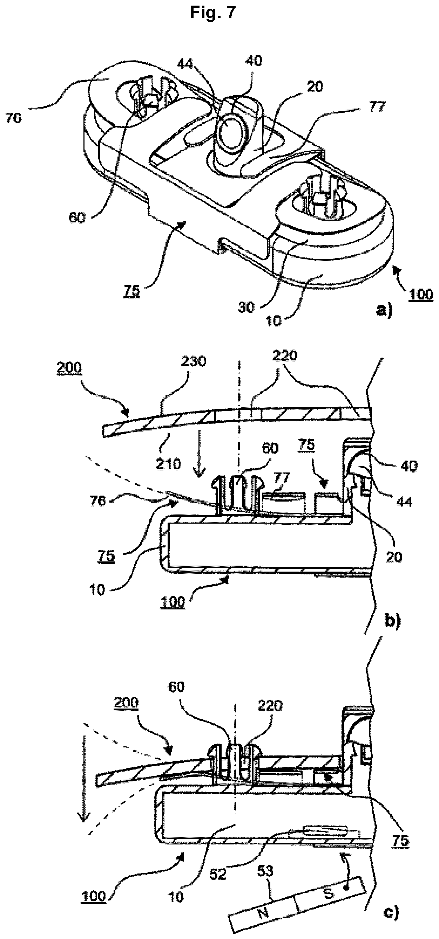

Fig. 7 zeigt eine Ausführungsvariante der Beleuchtungsvorrichtung **100** mit Befestigungsansätzen **60,** die als Schnapphaken ausgelegt sind und welche, wie in Fig. 7c 1 dargestellt, in Fensterungen **220** eines Sperrerblattes **200** einrasten. Fig. 7a) zeigt außerdem einen Federbügel **75,** welcher die gleiche Funktion annimmt wie die Membranhülle **70** in anderen Ausführungsformen. Der Federbügel **75** kann auf den Wandbereich **30** des Gehäuses **10** rastend, schnappend oder klebend aufgesetzt werden und besteht aus mehreren metallischen längs **76** und quer **77** verlaufenden Blattfedern, die sich beim Aufsetzen der Beleuchtungsvorrichtung **100** auf ein Sperrerblatt **200** der Ausformung der Oberfläche des Blattes entsprechend auslenken und anpassen und in einer angespannten Position verbleiben, so dass die Beleuchtungsvorrichtung **100** auch auf unebene Instrumentenoberflächen montiert werden kann. Der Federbüge! **75** gewährleistet einen festen Halt und verhindert ein Abbrechen des Zapfens und der Noppen bzw. der Befestigungsansätze, falls seitliche Kräfte, beispielsweise verursacht durch drückendes Gewebe, auf die Beleuchtungsvorrichtung wirken. Die angespannten Blattfedern **76, 77** wirken dann durch ihre zurückfedernden Eigenschaften und ihre vorgespannte Lagerung den auf die Beleuchtungsvorrichtung wirkenden Kräften entgegen. Die Ausführungsvariante der Befestigungsansätze **60** mit Schnapphaken verhindert, dass die Beleuchtungsvorrichtung sich durch den zurückfedernden Federbügel **75** wieder vom Sperrerblatt löst. Alternativ kann der Federbügel **75** auch so ausgelegt sein, dass er sich seitlich am Sperrerblatt **200** fixieren lässt und so die Beleuchtungsvorrichtung **100** zusätzlich einklemmt. In diesem Fall kann auf die Auslegung der Befestigungsansätze als Schnapphaken verzichtet werden. Die Aktivierung der Beleuchtungsvorrichtung erfolgt wiederum über einen internen Magnetschalter **52.**

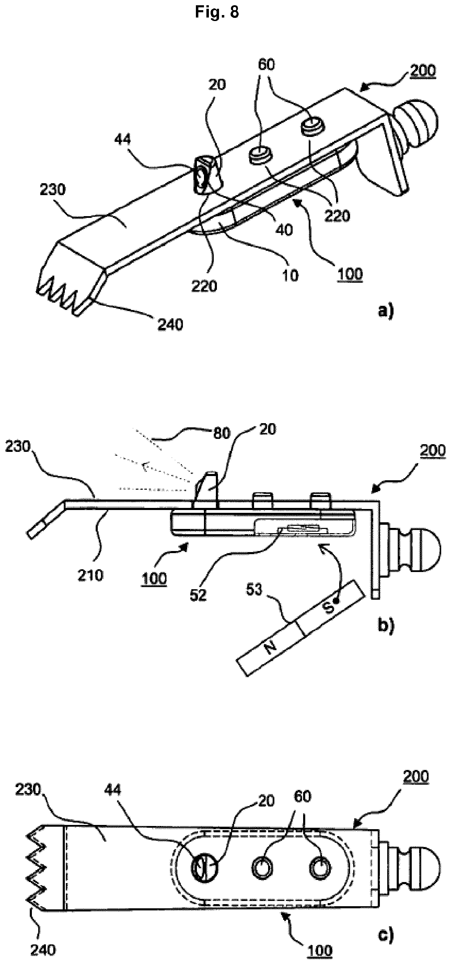

Fig. 8 zeigt ein weiteres Ausführungsbeispiel der Erfindung, bei dem eine etwas abgewandelte Beleuchtungsvorrichtung **100** an einem Sperrerblatt **200** mit einem abgekantetem, gezinkten unteren Abschnitt **240** befestigt ist. Die Beleuchtungsvorrichtung **100** ist derart abgewandelt, dass sich der Zapfen **20** an einem vorderen stirnseitigen Ende des Wandbereichs **30** des Gehäuses **10** befindet und ein Befestigungsansatz **60** und ein weiterer am hinteren Ende des Wandbereichs **30** des Gehäuses **10** angeordnet ist. Die Lichtaustrittsöffnung **40** weist hierdurch eine verbesserte Position zur Ausleuchtung des Operationsfeldes auf, denn sie ist so nah wie möglich zum Operationsfeld angeordnet. Die Wand **30** der Beleuchtungsvorrichtung **100** hat vollflächig Kontakt mit der Rückseite des Sperrerblatts **200;** die Vorrichtung **100** ist optimal an die längliche Form des Sperrerblatts angepasst. Die Beleuchtungsvorrichtung **100** ist an der oberen Blatthälfte des Sperrerblatts **200** befestigt. Hierdurch ist zwischen den Zinken des Abschnitts **240** und der Beleuchtungsvorrichtung **100** genügend Raum für das mit dem Sperrerblatt **200** zurückgehaltene Gewebe. Die Wirkung der Zinken wird durch die Anwesenheit des Gehäuses **10** nicht beeinträchtigt. Fig. 8b) zeigt eine Seitenansicht, in der angedeutet ist, dass die Aktivierung der Beleuchtungsvorrichtung magnetisch über einen externen Magneten **53** erfolgt.

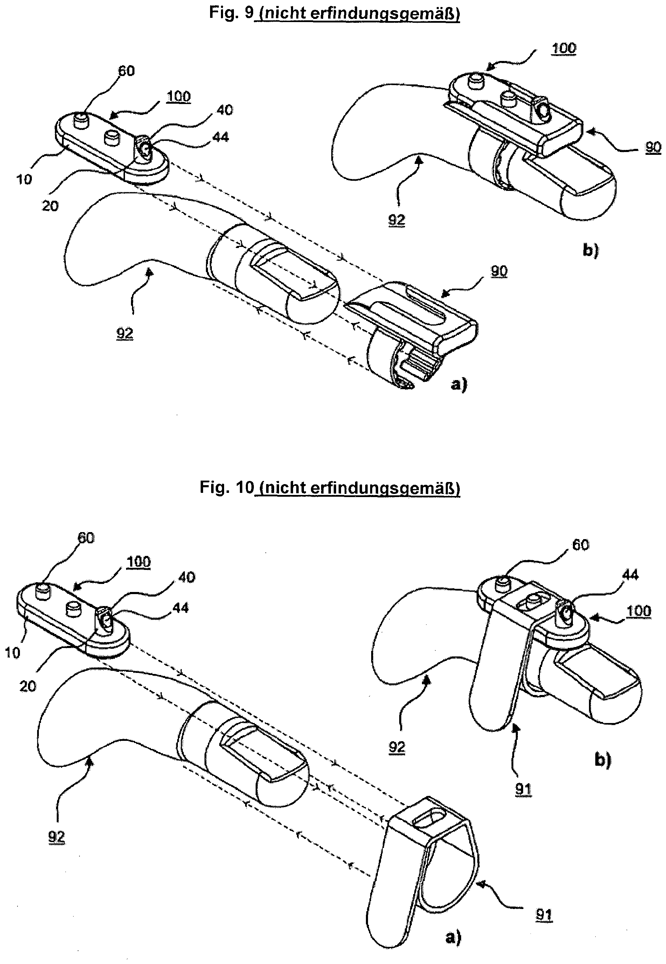

Fig. 9 eine nicht zur Erfindung gehörende Beleuchtungsvorrichtung in Kombination mit einem Adapterring **90** zum Aufstecken auf einen menschlichen Finger **92,** und zwar in Fig. 9a) in Explosionsdarstellung und in Fig. 9b) in aufgesetzter Position. Der Adapterring **90** ist wie ein Ring ausgebildet und kann auf einem Körperteil, z.B. auf einem Finger **92,** aber auch an verschiedensten Gegenständen befestigt werden. Der ringförmige Teil bzw. der Ringkörper trägt einen kastenförmigen Aufsatz, in den sich die Beleuchtungsvorrichtung hineinschieben und beispielsweise steckend fixieren lässt.

Der Ringkörper des Adapterrings **90** (Fig. 9a)) besitzt an seiner Unterseite eine schlitzartige Öffnung und ist aus einem dehnbaren Material hergestellt, so dass er sich an Finger **92** oder andere Körperteile mit verschiedenen Durchmessern anbringen lässt. Die wellige Ausformung der Innenseite des Ringkörpers, die am Finger **92** anliegt, dient ebenfalls der Adaption an unterschiedlich große Körperteile **92** und soll zusätzlich ein Abrutschen bzw. ein Abgleiten des Adapterringes **90** vom Körperteil **92** verhindern. Der kastenförmige Aufsatz des Adapterrings **90** dient lediglich der Aufnahme der Beleuchtungsvorrichtung.

Fig. 10 zeigt in entsprechender Darstellungsweise wie in Fig. 9 die nicht zur Erfindung gehörende Beleuchtungsvorrichtung in Kombination mit einem Adapterband **91,** welches um einen Finger **92** gewickelt wurde und per Klettverschluss befestigt wird. Das Adapterband **91** ist insbesondere ein Klettband mit passenden Öffnungen für die Aufnahme und die Fixierung der Beleuchtungsvorrichtung **100** an einem Körperteil **92.**

Fig. 11 zeigt eine bevorzugte Ausführungsform der Beleuchtungsvorrichtung **100** mit einem Zapfen **20** und zwei weiteren Befestigungsansätzen **60,** wobei ein Befestigungsansatz **60** gleichzeitig als Schalter ausgestaltet ist, um das Leuchtmittel einzuschalten und auszuschalten als auch optional dessen Lichtstärke (Helligkeit oder Helligkeitsstufen) zu regulieren. Der Zapfen **20** befindet sich in der Mitte, der fordere Befestigungsansatz **60** dient rein der Befestigung und der hintere Befestigungsansatz **60** ist als Druckschalter ausgestaltet, der gleichzeitig auch der Befestigung dient.

Fig. 12 zeigt eine bevorzugte Ausführungsform der Beleuchtungsvorrichtung **100** mit einem Zapfen **20** und zwei weiteren Befestigungsansätzen **60** und einer Solarzelle an der Unterseite des Gehäuses **10** der Beleuchtungsvorrichtung **100.** Die Solarzelle dient zur Aufladung eines Akkumulators oder einer Batterie innerhalb des Gehäuses **10,** der bzw. die dann wiederum als Stromquelle für das Leuchtmittel im Kopf des Zapfens **20** dient.

Die Figuren 13 bis 15 zeigen Ausführungsformen der Beleuchtungsvorrichtung **100** mit einem beweglichen Zapfen **20** und weiteren zwei Befestigungsansätzen **60.** Bei diesen Ausführungsformen ist einer der zwei zusätzlichen Befestigungsansätze **60** als Schalter ausgestaltet. Der Schalter dient zum Einschalten und Ausschalten des Leuchtmittels und weiter bevorzugt auch zur Einstellung der Helligkeit des Leuchtmittels. Bei diesen Ausführungsformen mit oder ohne als Schalter ausgestaltetem Befestigungsansatz **60** ist vorzugsweise in das Gehäuse **10** der Beleuchtungsvorrichtung **100** zumindest eine Solarzelle integriert, welche zur Aufladung eines Akkumulators oder einer Batterie innerhalb des Gehäuses **10** dient, wobei diese Aufladung im steril verpackten Zustand durch die Sterilverpackung hindurch erfolgen kann, ohne dass die steril verpackte Beleuchtungsvorrichtung **100** zum Aufladen entpackt und danach erneut sterilisiert werden muss.

### Technische Daten

Technische Daten der in den Figuren gezeigten Beispielbeleuchtungsvorrichtungen.

| **Geometrie** - **Maße und Gewicht** | |
|---|---|
| Höhe: | 8 mm |
| Länge: | 45 mm |
| Breite: | 14 mm |
| Gewicht: | 11 g |
| | |

| **Werkstoff** | |
|---|---|
| Material: | Biokompatibler Kunststoff |
| | |

| **Betrieb** | |
|---|---|
| Minimale Betriebszeit (nach 1. Jahr Lagerung bei 20°C): | 120 Minuten |
| | |

| **Energiequelle** | |
|---|---|
| Energiespeicher | Akkumulator |
| | |

| **Lichtparameter** | |
|---|---|
| Lichtquelle: | Leuchtdiode (SMD-Format) |
| Lichtfarbe: | Warmweiß |
| Lichtstärke: | 2.300 mcd |
| Beleuchtungswinkel: | 65° |
| Infrarotstrahlung: | keine |
| Farbtemperatur: | 5.000 K |
| CRI-Wert (Ra-Wert): | > 85 |
| | |

| **Mechanik** | |
|---|---|
| Befestigungsmechanismus: | Passung mit Übermaß |

| | |
|---|---|
| SMD: surface mounted device | |

### Bezugszeichenliste

- **100**: Beleuchtungsvorrichtung

- **10**: Gehäuse
- **20**: Zapfen
- **30**: Wandbereich
- **40**: Lichtaustrittsöffnung
- **44**: lichtdurchlässige Abdeckung / Linse
- **50**: Mechanischer Schalter (z.B. Druckschalter, Taster)
- **52**: Magnetschalter
- **53**: Magnet
- **60**: Befestigungsansatz (z. B. Noppe oder Schnapphaken)
- **70**: Membranhülle
- **71**: Fensterung Membranhülle
- **72**: Wandbereich Membranhülle
- **75**: Federbügel
- **76**: Längs-Blattfeder
- **77**: Quer-Blattfeder
- **80**: Lichtstrahl / Lichtkegel
- **90**: Adapterring
- **91**: Adapterband
- **92**: Körperteil (z. B. menschlicher Finger)

- **200**: chirurgisches Instrument (z. B. Sperrerblatt)
- **210**: Rückseite des Sperrerblattes
- **220**: Fensterung
- **230**: Vorderseite des Sperrerblattes
- **240**: gezinkter Abschnitt

## Patentansprüche

1. Beleuchtungsvorrichtung (100), die für die Befestigung an einem chirurgischen Instrument (200) oder über Adapter (90; 91) an einem Körperteil (92) eines Operateurs oder einer/s OP-Assistentin/-en, wie beispielsweise an einem Finger, ausgerüstet ist, um als Lichtquelle während einer chirurgischen Operation zur Ausleuchtung von Körper- und Organhöhlen zu dienen, mit einem Gehäuse (10) und wenigstens einem Leuchtmittel, wobei an dem Gehäuse (10) in einem in der Befestigungsposition zum chirurgischen Instrument gerichteten Wandbereich (30) wenigstens ein von der Wand des Gehäuses (10) nach außen weisender Zapfen (20) angeordnet ist, der an seinem distalen Ende eine Lichtaustrittsöffnung (40) besitzt und der dafür ausgelegt ist, eine Fensterung (220) des chirurgischen Instruments oder des Adapters (90; 91) zu durchgreifen und so mit der Fensterung eine form- oder kraftschlüssige Verbindung herzustellen und wobei an dem in der Befestigungsposition zum chirurgischen Instrument gerichteten Wandbereich (30) des Gehäuses (10) ein zusätzlicher Befestigungsansatz (60) in Form eines Stiftes oder einer Noppe vorgesehen ist, der zum Einschalten und Ausschalten des im Zapfen (20) angeordneten Leuchtmittels dient.

2. Beleuchtungsvorrichtung (100) nach Anspruch 1, **dadurch gekennzeichnet, dass** der Zapfen (20) ein im Wesentlichen zylindrischer Stift ist.

3. Beleuchtungsvorrichtung (100) nach Anspruch 1 oder 2, **dadurch gekennzeichnet**, das der Zapfen (20) biegbar und/oder drehbar ist.

4. Beleuchtungsvorrichtung (100) nach einem der Ansprüche 1 bis 3, **dadurch gekennzeichnet, dass** der in der Befestigungsposition zum chirurgischen Instrument (200) gerichtete Wandbereich (30) eben ist oder der Kontur des chirurgischen Instruments (200) in dem korrespondierenden Wandbereich (210) folgend geformt ist.

5. Beleuchtungsvorrichtung (100) nach einem der Ansprüche 1 bis 4, **dadurch gekennzeichnet, dass** an dem Wandbereich (30) des Gehäuses (10) wenigstens ein zusätzlicher Befestigungsansatz (60), insbesondere ein Stift, eine Noppe oder ein Schnapphaken, in Zuordnung zu einer passenden Aufnahme im chirurgischen Instrument vorgesehen ist.

6. Beleuchtungsvorrichtung (100) nach einem der Ansprüche 1 bis 5, **dadurch gekennzeichnet, dass** in das Gehäuse (10) eine Solarzelle integriert ist und/oder dass das Gehäuse (10) eine Energieversorgungseinheit enthält.

7. Beleuchtungsvorrichtung (100) nach einem der Ansprüche 1 bis 6, **dadurch gekennzeichnet, dass** die Beleuchtungsvorrichtung entweder über einen internen Magnetschalter (52) verfügt, der das Leuchtmittel durch Anlegen eines externen Magneten (53) oder eines Magnetfeldes aktiviert, oder einen per Hand betätigbaren mechanischen Schalter an seinem Gehäuse (10) trägt, oder einen Druckschalter oder Taster (50) besitzt, der so auf dem Wandbereich (30) des Gehäuses (10) angeordnet ist, dass er bei Befestigen der Beleuchtungsvorrichtung (100) am chirurgischen Instrument (200) betätigt wird.

8. Beleuchtungsvorrichtung (100) nach einem der Ansprüche 1 bis 7, **dadurch gekennzeichnet, dass** diese mit einer zusätzlichen Komponente ausgerüstet ist, nämlich mit einem Ausgleichselement (70) zwischen Wandbereich (30) des Gehäuses (10) und dem Wandbereich des chirurgischen Instruments (200; 210), um unebene Instrumentenoberflächen auszugleichen und die Festigkeit der Verbindung zwischen Instrument und Beleuchtungsvorrichtung zu erhöhen, oder mit einem Adapter (90; 91) für die Befestigung der Beleuchtungsvorrichtung (100) an einem Körperteil (92) eines Operateurs oder einer/s OP-Assistentin/-en, wie beispielsweise an einem Finger.

9. Set aus mindestens einer Beleuchtungsvorrichtung (100) nach einem der Ansprüche 1 bis 8 und mindestens einem chirurgischen Instrument, das für eine Verbindung mit der Beleuchtungsvorrichtung (100) nach einem der Ansprüche 1 bis 8 gefenstert ist.

10. Set nach Anspruch 9, **dadurch gekennzeichnet, dass** es wenigstens einen Adapter (90; 91) für die Fixierung der Beleuchtungsvorrichtung (100) an einem Körperteil oder ein Ausgleichselement für den Kontakt zwischen Wand (30) der Beleuchtungsvorrichtung (100) und Wand des Instruments (200) enthält.

## Claims

1. Lighting device (100) equipped for fastening to a surgical instrument (200) or by means of adapters (90, 91) on a body part (92) of a surgeon or an OP-assistant, for example on a finger, in order to serve as a light source during a surgical operation for the illumination of body and organ cavities, with a housing (10) and at least one illuminant, wherein at least one pin (20) pointing outwards from the wall of the housing (10) is arranged on the housing (10) in a wall area (30) directed towards the surgical instrument (200) in the fastening position, wherein the pin (20) has a light-emitting opening (40) at the distal end of the pin (20) and is designed for reaching through a fenestration (220) of the surgical instrument (200) or of the adapter (90; 91) and thus establishing a form-fit or force-fit connection with the fenestration and wherein an additional fastening projection (60) in form of stud or a nub is provided in the wall area (30), which is in its fastening position directed towards the surgical instrument (200), for switching the illuminant located in the pin (20) on and off.

2. Lighting device (100) according to claim 1, **characterized in that** the pin (20) is an essentially cylindrical stud.

3. Lighting device (100) according to claims 1 or 2, **characterized in that** the pin (20) is bendable and/or rotatable.

4. Lighting device (100) according to any one of claims 1 to 3, **characterized in that** the wall area (30) directed towards the surgical instrument (200) in the fastening position is plane or is shaped to follow the contour of the corresponding wall area (210) of the surgical instrument (200).

5. Lighting device (100) according to any one of claims 1 to 4, **characterized in that** on the wall area (30) of the housing (10) at least one additional fastening projection (60), in particular a stud, a nub or a snap-fit, is arranged in allocation to a suitable holder in the surgical instrument.

6. Lighting device (100) according to any one of claims 1 to 5, **characterized in that** a solar cell is integrated in the housing (10) and/or that the housing (10) contains an energy supply unit.

7. Lighting device (100) according to any one of claims 1 to 6, **characterized in that** the lighting device is either equipped with an integrated magnetic switch (52), which activates the illuminant via applying an external magnet (53) or a magnetic field, or bears a mechanical switch on its housing (10), which is operated manually, or has a press switch or button (50) that is arranged on the wall area (30) of the housing (10) in such a way, that it is activated when the lighting device (100) is fastened on the surgical instrument (200).

8. Lighting device (100) according to any one of claims 1 to 7, **characterized in that** it is equipped with an additional component, namely with a compensation element (70) inbetween the wall area (30) of the housing (10) and the wall area of the surgical instrument (200; 210), in order to balance uneven surfaces of the instrument and in order to increase the strength of the connection between the instrument and the lighting device, or with an adapter (90; 91) for the fastening of the lighting device (100) on a body part (92) of a surgeon or an OP-assistant, for example on a finger.

9. Set of at least one lighting device (100) according to any one of claims 1 to 8 and at least one surgical instrument, which is fenestrated for a connection with the lighting device (100) according to any one of claims 1 to 8.

10. A set according to claim 9, **characterized in that** it comprises at least an adapter (90, 91) for the fixation of the lighting device (100) to a body part or a compensation element for the contact between the wall (30) of the lighting device (100) and the wall of the instrument (200).

## Revendications

1. Dispositif d'éclairage (100) équipé pour être fixé sur un instrument chirurgical (200) ou sur une partie du corps à l'aide d'adaptateurs (90, 91), par exemple sur le doigt (92) d'un chirurgien ou d'un assistant d'opération, afin de servir comme source lumineuse durant une intervention chirurgicale, notamment à l'intérieur des cavités corporelles et organiques, comprenant un boîtier (10) et au moins une source lumineuse, dans lequel au moins une tige (20) est dirigée vers l'extérieur du boîtier (10) et est disposée sur la paroi (30) du boîtier (10) orientée vers l'instrument chirurgical (200) dans la position de fixation, dans lequel la tige (20) présente une sortie de lumière (40) à son extrémité distale et est conçue pour passer à travers une fenestration (220) de l'instrument chirurgical (200) ou de l'adaptateur (90, 91) établissant ainsi une connexion de forme ou de force avec la fenestration et dans lequel une saillie de fixation supplémentaire (60), sous forme de goujon ou de noeud, est prévue sur la paroi (30) orientée vers l'instrument chirurgical (200) dans la position de fixation, pour éteindre ou allumer la source lumineuse située dans la tige (20).

2. Dispositif d'éclairage (100) selon la revendication 1, **caractérisé par le fait que** la tige (20) est essentiellement un goujon cylindrique.

3. Dispositif d'éclairage (100) selon l'une des revendications précédentes 1 ou 2, **caractérisé par le fait que** la tige (20) est pliable et/ou rotative.

4. Dispositif d'éclairage (100) selon l'une quelconque des revendications précédentes 1 à 3, **caractérisé par le fait que** la paroi (30) orientée vers l'instrument chirurgical (200) dans la position de fixation, est plane ou façonnée de manière à suivre le contour de la paroi correspondante (210) de l'instrument chirurgical (200).

5. Dispositif d'éclairage (100) selon l'une quelconque des revendications précédentes 1 à 4, **caractérisé par le fait que** sur la paroi (30) du boîtier (10), est prévue au moins une saillie de fixation supplémentaire (60), en particulier un goujon, un noeud ou un clips, qui est disposée en allocation sur un support approprié dans l'instrument chirurgical.

6. Dispositif d'éclairage (100) selon l'une quelconque des revendications précédentes 1 à 5, **caractérisé par le fait qu'**une cellule solaire est intégrée dans le boîtier (10) et/ou que le boîtier (10) contient une unité d'alimentation en énergie.

7. Dispositif d'éclairage (100) selon l'une quelconque des revendications précédentes 1 à 6, **caractérisé par le fait qu'**il est équipé d'un interrupteur magnétique intégré (52), qui active la source lumineuse par l'application d'un aimant externe (53) ou d'un champ magnétique, ou qui comporte un interrupteur mécanique sur le boîtier (10), qui est actionné manuellement ou qui comporte un interrupteur ou un bouton (50) qui est disposé sur la paroi (30) du boîtier (10) de manière à être activé lorsque le dispositif d'éclairage (100) est fixé sur l'instrument chirurgical (200).

8. Dispositif d'éclairage (100) selon l'une quelconque des revendications précédentes 1 à 7, **caractérisé par le fait qu'**il est équipé d'un composant supplémentaire, à savoir un élément de compensation (70) entre la paroi (30) du boîtier (10) et la paroi de l'instrument chirurgical (200; 210), afin de compenser les surfaces irrégulières de l'instrument et de renforcer la connexion entre l'instrument et le dispositif d'éclairage, ou d'un adaptateur (90, 91) pour la fixation du dispositif d'éclairage (100) sur une partie du corps, par exemple sur le doigt (92) d'un chirurgien ou d'un assistant d'opération.

9. Set comprenant au moins un dispositif d'éclairage (100) selon l'une quelconque des revendications précédentes 1 à 8 et au moins un instrument chirurgical, dans lequel l'instrument chirurgical a une fenestration pour la connexion avec le dispositif d'éclairage (100) selon l'une quelconque des revendications précédentes 1 à 8.

10. Set selon la revendication 9, **caractérisé par le fait qu'**il comprend au moins un adaptateur (90, 91) pour la fixation du dispositif d'éclairage (100) sur une partie du corps ou un élément de compensation pour le contact entre la paroi (30) du dispositif d'éclairage (100) et la paroi de l'instrument (200).
